(19)

**Europäisches Patentamt**
**European Patent Office**
**Office européen des brevets**

(11) **EP 4 585 613 A1**

(12) **EUROPEAN PATENT APPLICATION**
published in accordance with Art. 153(4) EPC

(43) Date of publication:
**16.07.2025 Bulletin 2025/29**

(21) Application number: **23945587.6**

(22) Date of filing: **25.09.2023**

(51) International Patent Classification (IPC):
*C07K 14/78* (2006.01)    *C12N 1/21* (2006.01)
*C12N 15/12* (2006.01)    *C12N 15/70* (2006.01)
*A61L 27/50* (2006.01)    *A61L 15/32* (2006.01)
*A61K 47/42* (2017.01)    *A61K 38/39* (2006.01)

(86) International application number:
**PCT/CN2023/121059**

(87) International publication number:
**WO 2025/015705 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.07.2023 CN 202310883772**

(71) Applicant: **Shanxi Jinbo Bio-Pharmaceutical Co., Ltd.**
**Shanxi 030032 (CN)**

(72) Inventors:
• **ZHANG, Yongjian**
  **Taiyuan, Shanxi 030032 (CN)**
• **LAN, Xiaobin**
  **Taiyuan, Shanxi 030032 (CN)**
• **WANG, Lingling**
  **Taiyuan, Shanxi 030032 (CN)**
• **SONG, Haihong**
  **Taiyuan, Shanxi 030032 (CN)**
• **ZHANG, Xiaobo**
  **Taiyuan, Shanxi 030032 (CN)**
• **LIU, Xin**
  **Taiyuan, Shanxi 030032 (CN)**
• **YANG, Xia**
  **Taiyuan, Shanxi 030032 (CN)**
• **HE, Zhenrui**
  **Taiyuan, Shanxi 030032 (CN)**
• **ZHANG, Guoliang**
  **Taiyuan, Shanxi 030032 (CN)**

(74) Representative: **Cabinet Becker et Associés**
**25, rue Louis le Grand**
**75002 Paris (FR)**

Remarks:
The complete document including Reference Table(s) and the Sequence Listing(s) can be downloaded from the EPO website

(54) **METHOD FOR BIOSYNTHESIS OF HUMAN BODY STRUCTURAL MATERIAL TYPE-VIII COLLAGEN**

(57)    Provided is a method for biosynthesis of a human body structural material type-VIII collagen. Also provided is a collagen, comprising one or more repeating units, wherein the repeating units are connected directly or by means of linkers, and each repeating unit comprises an amino acid sequence selected from the group consisting of SEQ ID NO: 1, 4, 7, 10, 13, 16, 19, 22, 25 or 28, or a variant thereof. The collagen can be used for biological dressings, human body bionic materials or plastic surgery materials. According to the method, the type-VIII collagen is produced by utilizing a genetic engineering technology, thereby overcoming the defects in the prior art.

**EP 4 585 613 A1**

## Description

This application claims the priority of the Chinese invention patent application No: 202310883772.3 titled "METHOD FOR BIOSYNTHESIS OF HUMAN BODY STRUCTURAL MATERIAL TYPE-VIII COLLAGEN", filed on July 18, 2023. The content of aforementioned application is incorporated into this application by reference.

## Technical field

The present disclosure relates to the field of collagen, specifically to human structural material type VIII collagen and its biosynthesis method.

## Background of the Disclosure

Collagen is the most abundant and widely distributed protein in animals, and is the main component of connective tissue. Type VIII collagen is a short-chain non-fibrillar collagen and serves as the main component of the retinal membrane. Type VIII collagen is the main component of the membrane underlying the corneal endothelial cells. The membrane is basement membrane which separates corneal endothelial cells from the corneal stroma. Type VIII collagen is produced by endothelial cells and forms a unique hexagonal lattice structure. It exists around chondrocytes in the heart, brain, fiver, lungs and cartilage. In addition, type VIII collagen is also found around the actively proliferating blood vessels of brain tumors and in the large fibrotic blood vessels of vascular tumors.

Type VIII collagen, also known as short collagen or reticular collagen, consists of two similar alpha chains: $\alpha 1(VIII)$ and $\alpha 2(VIII)$. There are two homologous trimeric subtypes, $[\alpha 1(VIII)]3$ and $[\alpha 2(VIII)]3$, which are considered the main molecular types. However, heterotrimeric forms may also exist. The lamina elastica posterior of the bovine has a hexagonal network structure with thin type VIII collagen fibrils. Immunohistochemical analysis and electron microscopy observation indicate that type VIII collagen is the main component of the structural framework of this layer. In addition, through rotational shadow analysis, it is observed that type VIII collagen may form a tetrahedral supramolecular structure, which can provide structural support and regulate cell behavior.

Double knockout mice of the two type VIII collagen genes, Col8a1 and Col8a2, shows significant anterior segment hypoplasia phenotype, with spherical anterior chamber and corneal spherical protrusions. The corneal stroma shows diffuse thinning, similar to what is seen in human corneal globes. The lamina elastica posterior is significantly thinner and there is no anterior banded region. Corneal endothelial cells increase in size and decrease in number. Finally, the mutated corneal endothelial cells show a decrease in proliferation ability in response to different growth factors in vitro, indicating that type VIII collagen may act as an enhancer of growth factor induced cell proliferation.

Previously, type VIII collagen is described as a heterotrimer consisting of two $\alpha 1$ chains and one $\alpha 2$ chain, but in vitro studies have shown the formation of homologous trimers of either $\alpha 1$ or $\alpha 2$. These homotrimers are resistant to pepsin, and immunohistochemical studies have shown that they are not always co-located in the cornea, optic nerve, aorta, and umbilical cord. The total length of the $\alpha 1$ chain is 744 amino acids (aa), and the first 27 amino acids at the N-terminus are signal peptides.

The NC1 domain of type VIII collagen (aa 572-744) corresponds to a protein called statin, which has been shown to be an effective angiogenesis inhibitor with apoptotic-inducing activity on aortic endothelial cells. The $\alpha 2$ chain is 703 amino acids (aa) in length and has a signal peptide at its N-terminus from amino acids 1 to 28. The $\alpha 1$ and $\alpha 2$ procollagen genes each contain four exons. One of features of the genes is that the largest exon encodes the entire triple helix domain (COL1) and the C-terminal non-triple helix domain (NC1). Type VIII collagen has a high sequence homology with type X collagen and they have similar intron-exon structures. This indicates that the two types of collagen originate from the same precursor cell genes and belong to the same subclass of collagen. Type VIII collagen can form a hexagonal lattice structure like type X collagen.

Type VIII collagen is a glycoprotein that is exquisitely sensitive to neutrophil elastase. In contrast to other vascular collagens such as types I, II, IV, and V, it can be completely degraded within just 4 hours. Type VIII collagen is synthesized by aortic endothelial cells, corneal endothelial cells, pulmonary artery endothelial cells, and microvascular endothelial cells. Not all endothelial cells express type VIII collagen, as it is not present in either large or small blood vessels. In addition, both in vitro and in vivo, monocytes and macrophages are found to express type VIII collagen. Human mast cells have also been shown to produce type VIII collagen under normal and pathological conditions. It has been proven to contribute to angiogenesis, tissue remodeling, fibrosis, and cancer. Mast cells expressing type VIII collagen are found in the perivascular spaces. Type VIII collagen is also expressed in smooth muscle cells, stimulating cell migration.

The corneal endothelium secretes type VIII collagen, which is assembled into a hexagonal lattice structure within the endothelial membrane through the interaction of $\alpha 1$ and $\alpha 2$ polypeptides. COL8A2 gene mutation is associated with early Fuchs endothelial corneal dystrophy, but not with COL8A1. Type VIII collagen is believed to be involved in the differentiation and organization of endothelial cells. During heart development, type VIII collagen plays an important role in angiogenesis, as it is immunolocalized to the subendothelial layer of capillaries and arterioles. Type VIII collagen is up-regulated in early atherosclerosis and is thought to be associated with thrombosis and monocyte infiltration. It accumulates in atherosclerotic lesions, and its distribution pattern implies its role in plaque stability. Type VIII collagen is present around the blood vessels of actively proliferating brain tumors and in the large fibrotic blood vessels of hemangiomas. Finally, type VIII collagen is expressed in human diabetic nephropathy, but not in other kidney diseases.

To date, the vast majority of collagen used in various studies has come from animal tissues and skin extracts. Collagen extracted from animal tissues exhibits poor water solubility and weak processability, directly limiting the development of many potential applications. In contrast, collagen produced via recombinant DNA technology can effectively overcome these limitations.

## Summary of the Disclosure

The present disclosure is partially based on the following findings of the inventor: the inventor conducted large-scale functional region screening on human type VIII collagen and obtained different target gene functional regions of recombinant humanized type VIII collagen, among which, compared with the positive control (human type I collagen), have higher activity in promoting cell adhesion. The present disclosure also indicates that the collagen or polypeptide of the present disclosure is suitable for expression and purification. In addition, the inventor found that compared with other collagens (C8b and C8d-j), C8a and C8c have higher expression levels and better purification effects, and can be used for industrial production. C8a and C8c of the present disclosure also have a triple helix structure.

In one aspect, the present disclosure provides collagen or polypeptide comprising one or more repeating units linked directly or via a linker, wherein the repeating unit comprises an amino acid sequence selected from the group consisting of SEQ ID NO: 1, 4, 7, 10, 13, 16, 19, 22, 25, or 28, or a variant thereof, and wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence.

In one embodiment, the repeating units are 2-50 repeating units, such as 2-45, 2-40, 2-35, 2-30, 2-25, 2-20, 2-15, 2-10, or 2-8 repeating units.

In one embodiment, the linker comprises one or more amino acid residues, such as 1-10, 1-9, 1-8, 1-7, 1-6, 1-5, 1-4, 1-3, or 1-2 amino acid residues.

In one embodiment, the mutations are selected from a substitution, an addition, an insertion, or a deletion.

In one embodiment, the substitution is a conservative amino acid substitution.

In one embodiment, the collagen or the polypeptide is a recombinant collagen. In one embodiment, the collagen or the polypeptide is a recombinant type VIII collagen. In one embodiment, the collagen or the polypeptide is a human recombinant type VIII collagen.

In one polypeptide, the collagen or the polypeptide has cell adhesion activity or possesses a triple helix structure.

In one embodiment, the collagen or the polypeptide comprises an amino acid sequence selected from the group consisting of SEQ ID NO: 2, 5, 8, 11, 14, 17, 20, 23, 26, or 29, or a variant thereof, wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence.

In one embodiment, the mutations are selected from the group consisting of a substitution, an addition, an insertion or a deletion. In one embodiment, the substitution is a conservative amino acid substitution.

In another aspect, a nucleic acid is provided that encodes the collagen or the polypeptide as described herein. In one embodiment, the nucleic acid comprises a codon optimized nucleotide sequence. In one embodiment, a codon-optimized nucleotide sequence for expression in *Escherichia coli*. In one embodiment, the nucleic acid comprises a nucleotide sequence selected from the group consisting of SEQ ID NO: 3, 6, 9, 12, 15, 18, 21, 24, 27, or 30.

In another aspect, provided is a vector comprising a nucleic acid described herein. In one embodiment, the vector comprises an expression control element, nucleotides of a purification tag and/or nucleotides of a leader sequence operably linked to the nucleic acid. In one embodiment, the expression control element is selected from the group consisting of a promoter, a terminator or an enhancer. In one embodiment, the purification tag is selected from the group consisting of a His tag, a GST tag, an MBP tag, a SUMO tag or a NusA tag. In one embodiment, the vector is an expression vector or a cloning vector, preferably pET-28a (+). pET-28a (+) may comprise a HIS, Thrombin, or T7 protein tag at N-terminus, and an His tag at C-terminus. In the present disclosure, the collagen or polypeptide may comprise an enzymatic cleavage site at the N-terminus to facilitate purification, such as TEV enzymatic cleavage sites.

In another aspect, provided is a host cell comprising a nucleic acid or a vector described herein. In one embodiment, the host cell is a eukaryotic cell or a prokaryotic cell. In one embodiment, the eukaryotic cell is a yeast cell, an animal cell, and/or an insect cell. In one embodiment, the prokaryotic cell is an *Escherichia coli* cell, such as *E. coli* BL21.

EP 4 585 613 A1

In another aspect, provided is a composition comprising one or more of the collagens or polypeptides, nucleic acid, vector, and host cell described herein. In one embodiment, the composition is a kit. In one embodiment, the composition is one or more of a biological dressing, a human bionic material, a plastic surgery and beauty material, an organoid culture material, a cardiovascular stent material, a coating material, a tissue injection filling material, an ophthalmic material, an obstetrics and gynecology biomaterial, a nerve repair and regeneration material, a liver tissue material and blood vessel repair and regeneration material, a 3D printed artificial organ biomaterial, a cosmetic raw material, a pharmaceutical excipient, and a food additive. In one embodiment, the composition is a composition for injection use, or oral use.

In another aspect, provided is use of the collagen or polypeptide, the nucleic acid, the vector, the host cell, and/or the composition as described herein in one or more of a biological dressing, a human bionic material, a plastic surgery and beauty material, an organoid culture material, a cardiovascular stent material, a coating material, a tissue injection filling material, an ophthalmic material, an obstetrics and gynecology biomaterial, a nerve repair and regeneration material, a liver tissue material and blood vessel repair and regeneration material, a 3D printed artificial organ biomaterial, a cosmetic raw material, a pharmaceutical excipient, and a food additive.

In another aspect, provided is a method of promoting cell adhesion, comprising a step of contacting the collagen or polypeptide, the nucleic acid, the vector, the host cell, and/or the composition as described herein with a cell. In one embodiment, the cell is an animal cell. The animal cell may be a mammalian cell or a human cell.

In another aspect, provided is a method for performing plastic surgery or cosmetology, tissue injection filling, ophthalmic treatment, nerve repair, or vascular repair on a subject in need thereof, comprising administering the collagen or the polypeptide as described herein to the subject. In one embodiment, the administration is performed orally or by injection. In one embodiment, the subject has a disease or condition related to type VIII collagen deficiency, such as anterior segment hypoplasia.

In another aspect, provided is a method for producing the collagen or the polypeptide as described herein, comprising:

(1) culturing the host cell as described herein under a suitable culture condition;
(2) harvesting host cells and/or culture media containing the collagen or the polypeptide; and
(3) purifying the collagen or the polypeptide.

In one embodiment, the host cell is an E. coli cell, preferably an E. coli BL21 (DE3) cell.

In one embodiment, step (1) comprises culturing the E. coli cell in LB medium and inducing expression with IPTG.

In one embodiment, step (2) comprises harvesting *E. coli* bacterial cells, resuspending them in an equilibrium working solution, homogenizing the *E. coli* bacterial cells, preferably by high-pressure homogenization and isolating the supernatant. In one embodiment, the equilibrium working solution comprises 100-500 mM sodium chloride, 10-50 mM Tris, 10-50 mM imidazole, at pH 7-9.

In one embodiment, step (3) includes crude purification, enzymatic digestion, fine purification, and/or reverse nickel column purification. In one embodiment, step (3) includes crude purification, as well as one or more of enzymatic digestion, fine purification, and/or reverse nickel column purification.

In one embodiment, the crude purification includes purification of the supernatant by Ni-agarose gel column to obtain an eluant containing the target protein, wherein the eluant comprises 100-500mM sodium chloride, 10-50mM Tris and 100-500mM imidazole, preferably pH 7-9.

In one embodiment, the fine purification includes gradient elution of the enzymatically digested product using a strong anion exchange chromatography column. Preferably, gradient elution comprises 0-15% B solution for 1-5 minutes followed by maintaining 3 column volumes, 15-30% B solution for 1-5 minutes followed by maintaining 3 column volumes, 30-50% B solution for 1-5 minutes followed by maintaining 3 column volumes, and 50-100% B solution for 1-5 minutes followed by maintaining 3 column volumes. The B solution contains 10-50 mM Tris and 0.5-5 M sodium chloride, pH7-9.

In one embodiment, the reverse nickel column purification includes the purification of the enzymatically digested product on the Ni-agarose gel column. Preferably, the eluent comprises 10-50 mM Tris, 10-50 mM sodium chloride and 0.5-5M imidazole, pH7-9.

In this disclosure, the enzymatic digestion can be performed by TEV enzyme.

The advantages of the present disclosure include:

1. The collagens (such as C8a-C8j) of the present disclosure are derived from type VIII collagen and are a recombinant type VIII collagen. Specifically, the collagen is derived from human type VIII collagen and is a human recombinant type VIII collagen.

2. The collagens of the present disclosure (such as C8a-C8j) are suitable for production in *E. coli* and can be isolated and purified.

3. The collagens of the present disclosure (such as C8a and C8c) have a high expression level and are suitable for subsequent purification.

4. The collagens of the present disclosure have cell adhesion activity. The collagens of the present disclosure (such as C8a and C8c) exhibit higher cell adhesion activity compared to positive controls. The collagens of the present disclosure have a triple helix structure.

**Brief Description of the Drawings**

Fig. 1 shows the purification results of C8a. The yield of C8a is high, and the purity of the target protein is good after the fine purification.

Fig. 2 shows the purification results of C8b. The crude target protein of C8b contains some impurity bands. When washed with 1M solution, some target proteins are eluted. After fine purification, the yield is low, but the purity of the target protein is good.

Fig. 3 shows the purification results of C8c. The purity of the target protein of C8c is good. When digested with the enzyme at a ratio of 20:1, a small portion of the target protein is not digested.

Fig. 4 shows the purification results of C8d. The yield of C8d is relatively high, but the impurity band at 75kDa is not removed after reverse nickel column purification.

Fig. 5 shows the purification results of C8e. The crude target protein of C8e has impurity bands, and the yield is low.

Fig. 6 shows the purification results of C8f. The crude target protein of C8f has a relatively large number of impurity bands.

Fig. 7 shows the purification results of C8g. The yield of the crude C8g is relatively high, and the target protein appears as two bands.

Fig. 8 shows the purification results of C8h. The crude yield of C8h is relatively low.

Fig. 9 shows the purification results of C8i. For C8i, when digested with the enzyme at a ratio of 20:1, most of the protein is not cleaved, and even when the ratio is 5:1, it still remains undigested.

Fig. 10 shows the purification results of C8j. The crude yield of C8j is relatively low.

Fig. 11 shows the results of the cell adhesion activity detection of C8a and C8c.

Fig. 12 shows the circular dichroism scanning analysis result of C8a.

Fig. 13 shows the circular dichroism scanning analysis result of C8c.

**Detailed Embodiments**

In order to make the purpose, technical solution, and advantages of the present invention clearer, the following will give a clear and complete description of the technical solutions in the embodiments of the present invention in combination with these embodiments. Obviously, the described embodiments are part of the embodiments of the present invention, rather than all of them. Based on the embodiments of the present invention, all other embodiments obtained by those of ordinary skill in the art without inventive efforts shall fall within the scope of protection of the present invention.

Recombinant collagen is a new type of biomaterial screened and prepared by utilizing cutting-edge structural biology, genetic engineering, and other technologies with the genes encoding the functional regions of specific types of human collagen as templates, which has amino acid sequences that are identical or similar to those of human collagen.

As used herein, "type VIII collagen" is referred to as short collagen or reticular collagen. Type VIII collagen contains two similar $\alpha$ chains, $\alpha 1$ (VIII) and $\alpha 2$ (VIII), and two homologous trimeric subtypes, [$\alpha 1$ (VIII)] 3 and [$\alpha 2$ (VIII)] 3, which are considered the main molecular species, although heterotrimers may also exist. The lamina elastica posterior of the bovine has a hexagonal network structure with thin type VIII collagen fibrils. Immunohistochemical analysis and electron microscopy observation indicate that type VIII collagen is the main component of the structural framework of this layer. In addition, through rotational shadow analysis, it is observed that type VIII collagen may form a tetrahedral supramolecular structure, which can provide structural support and regulate cell behavior.

As used herein, "polypeptide" refers to multiple amino acid residues connected by peptide bonds. As used herein, a polypeptide contains one or more repeating units. The repeating units can be derived from human type VIII collagen. Thus, the polypeptide can be a human recombinant type VIII collagen. Multiple repeating units can be connected via a linker, and the linker can be a natural amino acid residue in human type VIII collagen from which the repeating unit is derives, for example, 1-50 amino acid residues. The repeating unit may be SEQ ID NO: 1, 4, 7, 10, 13, 16, 19, 22, 25 or 28. The polypeptide may be SEQ ID NO: 2, 5, 8, 11, 14, 17, 20, 23, 26 or 29.

As used herein, "human recombinant type VIII collagen" refers to a recombinant protein consisting of or substantially consisting of sequences derived from human type VIII collagen. As used herein, human recombinant type VIII collagen can consist of or substantially consist of fragments or multiple repeats of fragments derived from human type VIII collagen. As used herein, recombinant collagen, human recombinant type VIII collagen, collagen, or polypeptide can be used interchangeably.

As used herein, the term "variant" means a collagen or polypeptide having cell adhesion activity that includes alterations (i.e., substitutions, additions, insertions, and/or deletions) at one or more positions. Substitution means the replacement of an amino acid occupying a certain position with a different amino acid. Deletion means the removal of an amino acid occupying a certain position. Insertion means the addition of an amino acid adjacent to and immediately after an amino acid occupying a certain position. Addition refers to the addition of one or more amino acid residues to the C-terminus and/or N-terminus of an amino acid sequence. The substitution may be a conservative substitution. A variant of a repeating unit may be a sequence in which one or more amino acid residues in SEQ ID NO: 1, 4, 7, 10, 13, 16, 19, 22, 25 or 28 are changed or mutated (i.e., substituted, added, inserted and/or deleted). A variant of a collagen or polypeptide may be a sequence in which one or more amino acid residues in SEQ ID NO: 2, 5, 8, 11, 14, 17, 20, 23, 26 or 29 are changed or mutated (i.e., substituted, added, inserted and/or deleted).

In the context of the present disclosure, a conservative substitution may be defined by substitution within one or more of the amino acid classes reflected in one or more of the following tables:

Conservative classes of amino acid residues:

| | |
|---|---|
| Acidic residues | D and E |
| Basic residues | K, R and H |
| Hydrophilic uncharged residues | S, T, N, and Q |
| Aliphatic uncharged residues | G, A, V, L and I |
| Non-polar uncharged residues | C, M and P |
| Aromatic residues | F, Y, and W. |

Alternative physical and functional classification of amino acid residues:

| | |
|---|---|
| Alcohol group-containing residues | S and T |
| Aliphatic Residues | I, L, V and M |
| Cycloalkenyl-related residues | F, H, W and Y |
| Hydrophobic residues | A, C, F, G, H, I, L, M, R, T, V, W and Y |
| Negatively charged residues | D and E |
| Polar residues | C, D, E, H, K, N, Q, R, S and T |
| Positively charged residues | H, K and R |
| Small residues | A, C, D, G, N, P, S, T and V |
| Minimal residues | A, G and S |
| Residues involved in turn formation | A, C, D, E, G, H, K, N, Q, R, S, P and T |
| Flexible residues | Q, T, K, S, G, P, D, E, and R. |

As used herein, "cell adhesion" refers to the adhesion between cells and collagen. Collagen, such as the polypeptide described herein, can promote adhesion between cells and the container in which the cells are cultured.

As used herein, the term "expression" includes any step involved in the production of collagen or polypeptide, including, but not limited to, transcription, post-transcriptional modification, translation, post-translational modification, and secretion.

As used herein, the term "expression vector" means a linear or circular DNA molecule comprising a polynucleotide which encodes a collagen or polypeptide and is operably linked to a control sequence provided for its expression.

As used herein, the term "host cell" means any cell type amenable to transformation, transfection, transduction, etc. with a nucleic acid construct or expression vector comprising a polynucleotide of the present disclosure. The term "host cell" encompasses any progeny of a parent cell that is not identical to the parent cell due to a mutation occurring during replication.

As used herein, the term "nucleic acid" means a single-stranded or double-stranded nucleic acid molecule that is isolated from a naturally occurring gene, or is modified in a manner which is otherwise not present in nature to contain a segment of a nucleic acid, or is synthetic, and the nucleic acid molecule may comprise one or more control sequences. The nucleic acid may be SEQ ID NO: 3, 6, 9, 12, 15, 18, 21, 24, 27, or 30. The nucleic acid may be a codon-optimized nucleic acid, for example, a nucleic acid that is codon-optimized for expression in *E. coli* cells.

The term "operably linked" means a configuration in which a control sequence is placed at an appropriate position relative to an encoding sequence of a polynucleotide such that the control sequence directs the expression of the encoding sequence.

The degree of association between two amino acid sequences or between two nucleotide sequences is described by the parameter "sequence identity". For the purposes of this disclosure, the sequence identity between two amino acids is determined by Needleman-Wunsch Algorithm implemented by the Needle program (Needleman and Wunsch, 1970, J. Mol. Biol. 48: 443-453) of the EMBOSS software package (EMBOSS: The European Molecular Biology Open Software Suite, Rice et al, 2000, Trends Genet. 16: 276-277) (Version 5.0.0 or later is preferred). The parameters used are the gap opening penalty of 10, the gap extension penalty of 0.5, and the EBLOSUM62 (EMBOSS version of BLOSUM62) substitution matrix. Needle's output labeled "Longest Identity" (obtained using the non-simplified option) is used as the percent identity and calculated as follows:

(identical residues x 100) / (alignment length - total number of gaps in the alignment)

For the purposes of this disclosure, the sequence identity between two deoxynucleotide sequences is determined by the Needleman-Wunsch Algorithm implemented by the Needle program (Needleman and Wunsch, 1970, see supra) of the EMBOSS software package (EMBOSS: The European Molecular Biology Open Software Suite, Rice et al, 2000, see supra) (Version 5.0.0 or later is preferred). The parameters used are the gap opening penalty of 10, the gap extension penalty of 0.5, and the EDNAFULL (EMBOSS version of NCBI NUC4.4) substitution matrix. Needle's output labeled "Longest Identity" (obtained using the non-simplified option) is used as the percent identity and calculated as follows:

(identical deoxyribonucleotides x 100) / (alignment length - total number of gaps in the alignment)

**Collagen**

The present disclosure provides collagen or polypeptide comprising one or more repeating units linked directly or via a linker, wherein the repeating units comprise an amino acid sequence selected from the group consisting of SEQ ID NO: 1, 4, 7, 10, 13, 16, 19, 22, 25, or 28, or a variant thereof. The variant may be (1) an amino acid sequence in which one or more amino acid residues are mutated in the amino acid sequence of SEQ ID NO: 1, 4, 7, 10, 13, 16, 19, 22, 25, or 28, or (2) an amino acid sequence having at least 80%, 85%, 86%, 87%, 88%, 89%, 90%, 91%, 92%, 93%, 94%, 95%, 96%, 97%, 98%, 99%, or 100% identity to the amino acid sequence of SEQ ID NO: 1, 4, 7, 10, 13, 16, 19, 22, 25, or 28. With respect to the collagen or polypeptide described herein, the mutation may be selected from the group consisting of a substitution, addition, insertion, or deletion. Preferably, the substitution is a conservative amino acid substitution.

The collagen or the polypeptide described herein may comprise a plurality of repeating units, for example, 2-50 repeating units, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 repeating units.

The linker in collagen or the polypeptide described herein may contain one or more amino acid residues, such as 1-10, 1-9, 1-8, 1-7, 1-6, 1-5, 1-4, 1-3, or 1-2 amino acid residues.

The collagen or the polypeptide described herein is a recombinant collagen, especially a recombinant type VIII collagen, preferably with cell adhesion activity. The collagen or the polypeptide described herein can be derived from human, thus is a human recombinant type VIII collagen.

The recombinant collagen or polypeptide described herein may also comprise an amino acid sequence selected from the group consisting of SEQ ID NO: 2, 5, 8, 11, 14, 17, 20, 23, 26, or 29, or a variant thereof, wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence.

The collagen described herein can have a triple helix structure or three identical chains (i.e. trimeric form). The sequence of each chain can be the sequence of collagen or the polypeptide described herein.

**Nucleic acid constructs**

The present disclosure also relates to a nucleic acid construct comprising a nucleic acid of the present disclosure operably linked to one or more control sequences that direct the expression of an encoding sequence in a suitable host cell under a condition compatible with the control sequences. The vector may comprise a nucleic acid construct.

Nucleic acids can be modified in a variety of ways to enable the expression of collagen or polypeptide. Depending on the expression vector, it may be desirable or necessary to modify the nucleic acid prior to its insertion into the vector.

Techniques for modifying nucleic acids using recombinant DNA methods are well known in the art.

The control sequence may be a promoter, i.e., it is recognized by a host cell for the expression of a polynucleotide encoding a collagen or polypeptide of the present disclosure. The promoter comprises transcriptional control sequences that mediate the expression of collagen or polypeptide. The promoter can be any nucleic acid that exhibits transcriptional activity in a host cell, including variants, truncated or hybrid promoters, and can be obtained from a gene encoding an extracellular or intracellular collagen or polypeptide homologous or heterologous to the host cell.

Examples of suitable promoters for directing transcription of the vectors or nucleic acid constructs of the disclosure in bacterial host cells are promoters obtained from: *Bacillus amyloliquefaciens* alpha-amylase gene (amyQ), *Bacillus licheniformis* alpha-amylase gene (amyL), *Bacillus licheniformis* penicillinase gene (penP), *Bacillus stearothermophilus* maltoamylase gene (amyM), *Bacillus subtilis* fructan sucrase gene (sacB), Bacillus subtilis xylA and xylB genes, *Bacillus thuringiensis* cryIIIA gene, *E. coli* lac operon, and *E. coli* trc promoter.

In yeast hosts, useful promoters are obtained from the following genes: *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triosephosphate isomerase (TPI), *Saccharomyces cerevisiae* metallothionein (CUP1), and *Saccharomyces cerevisiae* 3-phosphoglycerate kinase.

The control sequence may also be a transcription terminator recognized by the host cell to terminate transcription. The terminator may be operably linked to the 3' end of the polynucleotide encoding the collagen or polypeptide. Any terminator that is functional in the host cell may be used in the present disclosure.

Preferred terminators for bacterial host cells are obtained from the genes of *Bacillus clausii* alkaline protease (aprH), *Bacillus licheniformis* alpha-amylase (amyL), and *E. coli* ribosomal RNA (rrnB).

Preferred terminators for yeast host cells are obtained from the following genes: *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al. (1992, see supra).

The control sequence may also be an mRNA stabilizer region downstream of the promoter and upstream of the encoding sequence of the gene, which increases the expression of the gene.

Examples of suitable mRNA stabilizer regions are obtained from the following genes: *Bacillus thuringiensis* cryIIIA gene (WO 94/25612) and *Bacillus subtilis* SP82 gene (Hue et al., 1995, Journal of Bacteriology 177: 3465-3471).

The control sequence may also be a leader sequence, i.e., a non-translated region of an mRNA that is important for translation in the host cell. The leader sequence is operably linked to the 5' end of the polynucleotide encoding the collagen or polypeptide. Any leader sequence that is functional in the host cell may be used.

Suitable leader sequences for yeast host cells are obtained from the following genes: Saccharomyces cerevisiae enolase (ENO-1), Saccharomyces cerevisiae 3-phosphoglycerate kinase, Saccharomyces cerevisiae alpha-factor, and Saccharomyces cerevisiae alcohol dehydrogenase/glyceraldehyde-3-phosphate dehydrogenase (ADH2/GAP).

The control sequence may also be a polyadenylation sequence, which is operably linked to the 3' end of the polynucleotide and when transcribed, is recognized by the host cell as a signal to add polyadenylate residues to the transcribed mRNA. Any polyadenylation sequence that is functional in the host cell may be used.

Useful polyadenylation sequences for yeast host cells are described by Guo and Sherman, 1995, Mol. Cellular Biol. 15: 5983-5990.

The control sequence may also be a signal peptide encoding region that encodes a signal peptide linked to the N-terminus of the collagen or polypeptide and directs the collagen or polypeptide into the secretory pathway of the cell. The 5'-end of the encoding sequence of the polynucleotide may itself contain a signal peptide encoding sequence that is naturally linked in open reading frame to the segment of the encoding sequence encoding the collagen or polypeptide. Alternatively, the 5'-end of the encoding sequence may contain a signal peptide encoding sequence that is foreign to the encoding sequence. In cases where the encoding sequence does not naturally contain a signal peptide encoding sequence, a foreign signal peptide encoding sequence may be required. Alternatively, the foreign signal peptide encoding sequence may simply replace the natural signal peptide encoding sequence in order to enhance the secretion of the collagen or polypeptide. However, any signal peptide encoding sequence that directs the expressed collagen or polypeptide into the secretory pathway of the host cell may be used.

An effective signal peptide encoding sequence for a bacterial host cell is a signal peptide encoding sequence obtained from the following genes: *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral protease (nprT, nprS, nprM) and *Bacillus subtilis* prsA. Additional signal peptides are described by Simonen and Palva, 1993, Microbiological Reviews 57: 109-137.

Useful signal peptides for yeast host cells are obtained from the following genes: Saccharomyces cerevisiae alpha-factor and Saccharomyces cerevisiae invertase. Other useful signal peptide encoding sequences are described by Romanos et al. (1992, see supra).

**Expression vector**

The disclosure also relates to recombinant expression vectors comprising a nucleic acid, a promoter, and transcription and translation termination signals of the present disclosure. Nucleic acids and control sequences may be ligated together to produce recombinant expression vectors that may include one or more convenient restriction sites for insertion or substitution of a polynucleotide encoding the collagen or polypeptide at such sites. Alternatively, the polynucleotide may be expressed by inserting a nucleic acid, or a nucleic acid construct comprising the nucleic acid into an appropriate vector for expression. When an expression vector is produced, the encoding sequence is located in the vector such that the encoding sequence is operably linked to an appropriate control sequence for expression.

A recombinant expression vector may be any vector (e.g., a plasmid or virus) that can be conveniently subjected to a recombinant DNA procedure and may enable the expression of a polynucleotide. The choice of the vector will typically depend on the compatibility of the vector with the host cell into which the vector is to be introduced. The vector may be a linear chain or a closed circular plasmid.

The vector may be an autonomously replicating vector, i.e. a vector that exists as an extrachromosomal entity, whose replication is independent of chromosomal replication, such as a plasmid, an extrachromosomal element, a minichromosome or an artificial chromosome. The vector may contain any means for ensuring self-replication. Alternatively, the vector may be one that, when introduced into a host cell, integrates into the genome and replicates with one or more chromosomes into which it has been integrated. Furthermore, separate vectors or plasmids or two or more vectors or plasmids, which collectively contain the total DNA to be introduced into the genome of the host cell may be used, or transposons may be used.

The vector preferably contains one or more selectable markers that allow for convenient selection of transformed cells, transfected cells, transduced cells and the like. A selectable marker is a gene whose product provides a biocide resistance or a virus resistance, a resistance to heavy metals, a prototrophy to auxotroph, etc.

Examples of bacterial selectable markers are the dal gene from Bacillus licheniformis or Bacillus subtilis, or markers conferring antibiotic resistance, such as ampicillin, chloramphenicol, kanamycin, neomycin, spectinomycin, or tetracycline resistance. Suitable markers for yeast host cells include, but are not limited to: ADE2, HIS3, LEU2, LYS2, MET3, TRP1, and URA3.

The selectable marker may be a dual selectable marker system as described in WO 2010/039889. In one aspect, the dual selectable marker is an hph-tk dual selectable marker system.

The vector may contain an element that allows integration of the vector into the genome of the host cell or autonomous replication of the vector in the cell independent of the genome.

For integration into the host cell genome, the vector may rely on the polynucleotide sequence encoding the collagen or polypeptide or any other element of the vector for integration into the genome by homologous or non-homologous recombination. Alternatively, the vector may contain an additional polynucleotide for directing integration by homologous recombination into the genome of the host cell at a precise location(s) in the chromosome(s). To increase the likelihood of integration at a precise location, the integrational element should contain a sufficient number of nucleic acids, such as 100 to 10,000 base pairs, 400 to 10,000 base pairs, and 800 to 10,000 base pairs, which have a high degree of sequence identity to the corresponding target sequences to enhance the probability of homologous recombination. The integrational element may be any sequence homologous to a target sequence within the genome of the host cell. Furthermore, the integrational element may be a non-encoding or encoding polynucleotide. In another aspect, the vector can be integrated into the genome of the host cell by non-homologous recombination.

In order to replicate autonomously, the vector may further comprise an origin of replication enabling the vector to replicate autonomously in the host cell in question. The origin of replication may be any plasmid replicator that functions in cells to mediate autonomous replication. The term "origin of replication" or "plasmid replicator" means a polynucleotide that enables a plasmid or vector to replicate in vivo.

Examples of bacterial origins of replication are the origins of replication of plasmids pBR322, pUC19, pACYC177, and pACYC184 that allow replication in E. coli, as well as the origins of replication of plasmids pUB 110, pE194, pTA1060, and pAMβ1 that allow replication in Bacillus.

Examples of origins of replication for use in yeast host cells are 2-micron origin of replication, ARS1, ARS4, the combination of ARS1 and CEN3, as well as the combination of ARS4 and CEN6.

More than one copy of the polynucleotide of the present disclosure can be inserted into a host cell to increase the production of collagen or polypeptide. An increased number of copies of a polynucleotide can be obtained by integrating at least one additional copy of the sequence into the host cell genome or by including an amplifiable selectable marker gene together with the polynucleotide where cells comprising amplified copies of the selectable marker gene and thus additional copies of the polynucleotide can be selected by culturing the cells in the presence of an appropriate selectable agent.

Procedures for ligating the elements described above to construct the recombinant expression vectors of the present disclosure are well known to those of ordinary skill in the art (see, for example, Sambrook et al., 1989).

**Host cell**

The present disclosure also relates to recombinant host cells comprising a polynucleotide of the present disclosure operably linked to one or more control sequences that direct the production of the collagen or polypeptide of the present disclosure. The construct or vector comprising the polynucleotide is introduced into the host cell such that the construct or vector is maintained as a chromosomal integrant or as an autonomously replicating extrachromosomal vector, as described earlier. The term "host cell" encompasses any progeny of a parent cell that is not identical to the parent cell due to a mutation occurring during replication. The choice of host cell will depend largely on the gene encoding the collagen or polypeptide and its source.

The host cell may be any cell useful in the recombinant production of the collagen or polypeptide of the present disclosure, for example, a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, Escherichia coli, Flavobacterium, Fusobacterium, Helicobacter, Ilyobacter, Neisseria, Pseudomonas, Salmonella,* and *Ureaplasma.*

The host cell may also be a cell of a eukaryotic organism, such as a mammalian, insect, plant or fungus.

The host cells may be fungal cells such as *Basidiomycota, Chytridiomycota, Zygomycota,* and *Oomycota,* among others. The host cell may be a yeast cell, including *ascosporogenous* yeast (*Endomycetales*)*, basidiosporogenous* yeast, and yeast belonging to *Fungi Imperfecti* (*Blastomycetes*). The yeast host cell may be a cell of *Candida, Hansenula, Kluyveromyces, Pichia, Saccharomyces, Schizosaccharomyces* or *Yarrowia,* such as a cell of *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis* or *Yarrowia lipolytica.*

**Production method**

The present disclosure also relates to a method of producing the collagen or polypeptide as described herein, comprising:

(1) culturing the host cell described herein under a suitable culture condition;
(2) harvesting the host cell and/or culture medium comprising the collagen or polypeptide; and
(3) purifying the collagen or polypeptide.

Using methods known in the art, the host cells are cultured in a suitable nutrient medium for the production of collagen or polypeptide. For example, cells can be cultured in shake flask, or by small- or large-scale fermentation (including continuous, batch, fed-batch or solid-state fermentation) in a laboratory or industrial fermenter, wherein the cultivation is performed in a suitable medium and under conditions that allow expression and/or isolation of the collagen or polypeptide. The cultivation is performed in a suitable nutrient medium comprising carbon and nitrogen sources and inorganic salts using procedures known in the art. Suitable media may be obtained from commercial suppliers or may be prepared according to disclosed compositions (e.g., in the catalogs of American Type Culture Collection). If a collagen or polypeptide collagen is secreted into the nutrient medium, the collagen or polypeptide can be recovered directly from the medium. If a collagen or polypeptide is not secreted, it can be recovered from the cell lysate.

The collagen or polypeptide can be detected using methods known in the art that are specific for the collagen or polypeptide. These detection methods include, but are not limited to, the use of specific antibodies, formation of a product under an enzyme or disappearance of a substrate under an enzyme. For example, an enzymatical assay can be used to determine the activity of the collagen or polypeptide.

The collagen or polypeptide can be recovered by methods known in the art. For example, the collagen or polypeptide can be recovered from the nutrient medium by conventional procedures including, but not limited to, collection, centrifugation, filtration, extraction, spray drying, evaporation, or precipitation. In one aspect, the collagen or polypeptide is recovered from the fermentation broth comprising the collagen or polypeptide.

The collagen or polypeptide can be purified by known procedures in the art, including, but not limited to, chromatography (e.g., ion exchange chromatography, affinity chromatography, hydrophobic chromatography, focusing chromatography, and size exclusion chromatography), electrophoretic procedures (e.g., preparative isoelectric focusing electrophoresis), differential solubility (e.g., ammonium sulfate precipitation), SDS-PAGE, or extraction to obtain substantially pure collagen or polypeptide.

Step (1) may comprise one or more of the following steps: constructing an expression plasmid, e.g., inserting an encoding nucleotide sequence into a pET-28a-Trx-His expression vector to obtain a recombinant expression plasmid. Successfully constructed expression plasmids can be transformed into *E. coli* cells (e.g., *E. coli* competent cells BL21 (DE3)). The specific process can be as follows: (1) taking the plasmid to be transformed and adding it to *E. coli* competent cells BL21 (DE3); (2) placing the mixture on ice in an ice bath (e.g., for 10-60 min, e.g., 30 min), followed by a heat shock in a water bath (e.g., at 40-50°C, e.g., 42°C, for 45-90 s), taking out the mixture and placing it on ice in an ice bath (e.g., for 1-5 min, e.g., 2 min); (3) adding liquid LB medium followed by an incubation (e.g., at 35-40°C, e.g., 37°C, at 150-300 rpm, e.g., 220 rpm for 40-80 min, e.g., 60 min); (4) spreading the bacterial solution and picking single colonies. For example, the bacterial solution is taken and evenly spread on LB plates containing ampicillin sodium, and the plates are cultured in an incubator at 37°C for 15-17 hours until uniform-sized colonies grow in the plates.

Step (2) may comprise culturing the single colonies in LB medium containing an antibiotic stock (e.g., in a shaker at 150-300 rpm, e.g., 220 rpm, at a constant temperature of 35-40°C, e.g., 37°C for 5-10 hours, e.g., 7 hours). Then, the cultured shake flask is cooled to 10-20°C, for example 16°C, and IPTG is added to induce expression for a period of time, and then the cells are collected (for example, by centrifugation).

Step (3) may include resuspending the bacterial cells with an equilibrium working solution, cooling the bacterial liquid to ≤ 15°C, performing homogenization (e.g., high-pressure homogenization, e.g., 1-5 times, e.g., 2 times), and separating the homogenized bacterial liquid to obtain a supernatant. The equilibrium working solution may comprise 100-500 mM sodium chloride, 10-50 mM Tris, and 10-50 mM imidazole, at pH 7-9. For example, the concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The concentration of Tris may be 10, 15, 20, 25, 30, 35, 40, 45, or 50 nM. The concentration of imidazole may be 10, 15, 20, 25, 30, 35, 40, or 50 nM. The pH may be 7, 7.5, 8, 8.5, or 9.

Step (3) may include purifying and enzymatically digesting the collagen or polypeptide. The purification may be a crude purification comprising a Ni-agarose column purification of the supernatant to obtain an eluate containing the target protein. Crude purification may comprise washing the column material with water, for example for 2-10 column volumes (CVs), for example 5 CVs. The column material can be equilibrated with an equilibration solution (200 mM sodium chloride, 25 mM Tris, 20 mM imidazole at pH 8.0), for example for 2-10 CVs, for example 5 CVs. The equilibrium solution may comprise 100-500 mM sodium chloride, 10-50 mM Tris, and 10-50 mM imidazole at pH 7-9. For example, the concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The concentration of Tris may be 10, 15, 20, 25, 30, 35, 40, 45, or 50 nM. The concentration of imidazole may be 10, 15, 20, 25, 30, 35, 40, 45, or 50 nM. The pH may be 7, 7.5, 8, 8.5, or 9.

Step (3) may comprise loading the supernatant onto the column material and washing the impurity proteins with a washing solution. The washing solution may comprise 100-500 mM sodium chloride, 10-50 mM Tris, and 10-50 mM imidazole at pH 7-9. For example, the concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The concentration of Tris may be 10, 15, 20, 25, 30, 35, 40, 45, or 50 nM. The concentration of imidazole may be 10, 15, 20, 25, 30, 35, 40, 45, or 50 nM. The pH may be 7, 7.5, 8, 8.5, or 9. Then, the eluent can be added and the flow-through liquid is collected. The eluent may comprise 100-500 mM sodium chloride, 10-50 mM Tris, 100-500 mM imidazole, at pH 8.0. For example, the concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The concentration of Tris may be 10, 15, 20, 25, 30, 35, 40, 45, or 50 nM. The concentration of imidazole may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The pH may be 7, 7.5, 8, 8.5, or 9.

The enzymatic digestion may comprise adding a TEV protease for enzymatic digestion (at a ratio of the total amount of protein to the total amount of TEV protease of 10-100: 1, e.g., 50: 1, at 10-20°C, e.g., 16°C for 2-8 h, e.g., 4 h). The enzymatically digested protein solution is dialyzed, for example, in a dialysis bag at 1-6°C, for example 4°C for 1-8 hours, for example 2 hours, and then transferred to a new dialysate for dialysis at 1-6°C, for example 4°C overnight.

Purification may include fine purification (e.g., for proteins with an isoelectric point > 8.0). Preferably, the fine purification comprises using a strong anion exchange chromatography column (e.g., the pH may be 7, 7.5, 8, 8.5 or 9.) The eluate containing the target protein or the product after enzymatic digestion (e.g., enzymatically digested and dialyzed product) is subjected to gradient elution. The gradient elution comprises 0-15% solution B for 1-5 minutes followed by holding for 1-5 column volumes, e.g., 3 column volumes, 15-30% solution B for 1-5 minutes followed by holding for 1-5 column volumes, e.g., 3 column volumes, 30-50% solution B for 1-5 minutes followed by holding for 1-5 column volumes, e.g., 3 column volumes, 50-100% solution B for 1-5 minutes followed by holding for 1-5 column volumes, e.g., 3 column volumes. Solution B may comprise 10-50 mM Tris, 0.5-5 M sodium chloride, at pH 7-9. For example, the concentration of Tris is 15, 20, 25, 30, 35, 40, or 45 mM. The concentration of sodium chloride is 1, 2, 3, or 4 M. The pH may be 7, 7.5, 8, 8.5, or 9. Fine purification may include equilibrating the column material with solution A and loading, followed by gradient elution. Solution A may contain 10-50 mM Tris, 10-50 mM sodium chloride, at pH 7-9. For example, the concentration of Tris is 15, 20, 25, 30, 35, 40, or 45 mM. The concentration of sodium chloride is 15, 20, 25, 30, 35, 40 or 45 mM. The pH may be 7, 7.5, 8, 8.5, or 9.

Purification can include reverse nickel column purification (e.g. protein isoelectric point<8.0). The reverse nickel column purification can include the purification of the products after digestion (such as the products after dialysis) on the Ni-agarose gel column. The eluent may contain 10-50 mM (e.g. 15, 20, 25, 30, 35, 40, or 45 mM) Tris, 10-50 mM (e.g. 15, 20, 25, 30, 35, 40, or 45 mM) sodium chloride, 0.5-5 mM (e.g. 1, 2, 3, or 4 mM) imidazole, pH 7-9 (e.g. 7, 7.5, 8, 8.5, or 9).

Further provide the following examples to illustrate the present disclosure.

**Examples**

The present disclosure is further illustrated by the following examples, but any example or combination thereof should not be construed as limiting the scope of the present disclosure. The scope of the present disclosure is defined by the following claims. As a person skilled in the art combines this specification with common knowledge in the art, they can clearly discern the scope defined by the claims. Without departing from the spirit and scope of the present disclosure, those skilled in the art can make modifications to the technical solution of the present disclosure. Such modifications also fall within the scope of the present disclosure.

**Example 1: Construction, Expression, and Screening of Type VIII Collagen Fragments**

1. Large-scale screening for functional regions was carried out to obtain the following different target gene functional regions of recombinant type VIII humanized collagen.

1) C8a amino acid sequence:

gKpgmpgmpgKpgamgmpgaKgEigqKgEigpmgipgpqgppgphglp (SEQ ID NO: 1)
gKpgmpgmpgKpgamgmpgaKgEigqKgEigpmgipgpqgppgphglp
gKpgmpgmpgKpgamgmpgaKgEigqKgEigpmgipgpqgppgphglp
gKpgmpgmpgKpgamgmpgaKgEigqKgEigpmgipgpqgppgphglp

gKpgmpgmpgKpgamgmpgaKgEigqKgEigpmgipgpqgppgphglp
gKpgmpgmpgKpgamgmpgaKgEigqKgEigpmgipgpqgppgphglp

(The amino acid sequence of the repeat unit of C8a is shown as SEQ ID NO: 1, with a repeat number of 6; the amino acid sequence of C8a is shown as SEQ ID NO: 2.)

Nucleotide sequence:

GGGAAACCCGGAATGCCGGGCATGCCGGGGAAGCCGGGTGCGATGGGTATGCCGGGCG
CGAAAGGCGAGATCGGCCAGAAGGGCGAAATTGGCCCGATGGGTATTCCGGGTCCGCA
GGGTCCACCGGGTCCGCATGGTCTGCCGGGCAAGCCGGGTATGCCGGGCATGCCGGGTA
AGCCGGGTGCAATGGGTATGCCGGGCGCGAAAGGTGAAATCGGCCAAAAAGGTGAGAT
CGGCCCGATGGGTATTCCGGGTCCGCAGGGTCCTCCGGGCCCGCACGGCCTGCCTGGTA
AACCGGGTATGCCCGGCATGCCGGGCAAGCCGGGTGCAATGGGTATGCCGGGCGCGAA
AGGTGAGATCGGTCAAAAAGGTGAAATTGGTCCGATGGGCATTCCGGGTCCGCAGGGC
CCACCGGGTCCGCACGGCCTGCCGGGTAAGCCGGGTATGCCGGGGATGCCAGGCAAGC
CAGGTGCGATGGGTATGCCGGGCGCGAAAGGTGAAATTGGTCAGAAAGGTGAGATCGG
TCCGATGGGTATTCCGGGTCCGCAAGGTCCGCCTGGTCCGCATGGCTTGCCGGGCAAGC
CGGGTATGCCGGGCATGCCGGGCAAGCCGGGCGCTATGGGAATGCCTGGCGCGAAAGG
TGAGATCGGACAAAAAGGCGAAATCGGTCCGATGGGCATCCCGGGCCCACAGGGTCCA
CCGGGTCCCCACGGCCTGCCGGGTAAGCCGGGCATGCCGGGCATGCCGGGCAAGCCGG
GCGCTATGGGTATGCCGGGTGCGAAAGGTGAAATCGGCCAGAAGGGCGAGATCGGTCC
GATGGGTATTCCGGGCCCACAAGGTCCGCCGGGCCCACACGGTTTACCG (SEQ ID NO: 3)

2) C8b amino acid sequence:

gKpggpglpgqpgpKgDRgpKglpgpqglRgpKgDK (SEQ ID NO: 7)
gKpggpglpgqpgpKgDRgpKglpgpqglRgpKgDK
gKpggpglpgqpgpKgDRgpKglpgpqglRgpKgDK
gKpggpglpgqpgpKgDRgpKglpgpqglRgpKgDK
gKpggpglpgqpgpKgDRgpKglpgpqglRgpKgDK
gKpggpglpgqpgpKgDRgpKglpgpqglRgpKgDK

(The amino acid sequence of the repeat unit of C8b is shown as SEQ ID NO: 7, with a repeat number of 6; the amino acid sequence of C8b is shown as SEQ ID NO: 8.)

Nucleotide sequence:

GGGAAACCCGGAGGACCGGGTCTGCCGGGCCAACCGGGTCCGAAGGGCGACCGCGGT
CCGAAGGGTCTGCCGGGCCCACAGGGTCTGCGTGGTCCAAAAGGCGATAAAGGCAAAC
CGGGTGGTCCGGGCCTTCCGGGCCAGCCTGGCCCAAAGGGCGATCGTGGTCCGAAGGG
CTTGCCGGGCCCACAGGGTCTGCGTGGTCCGAAGGTGACAAGGGCAAACCGGGCGGT
CCGGGCCTGCCGGGGCAACCTGGCCCCAAGGGCGACCGCGGTCCGAAGGGTCTGCCGG
GTCCGCAAGGTCTGCGCGGTCCGAAGGGTGATAAAGGCAAACCGGGTGGTCCGGGCCT
GCCAGGCCAGCCGGGTCCGAAGGGCGATCGTGGCCCGAAGGGCTTGCCGGGTCCGCAG
GGTTTGAGAGGCCCAAAGGGCGACAAAGGTAAACCGGGCGGTCCGGGCCTGCCGGGT
CAGCCGGGTCCGAAGGGTGATCGTGGTCCGAAAGGCCTCCCGGGCCCTCAAGGTCTGC
GTGGTCCGAAGGGCGACAAAGGTAAACCGGGCGGTCCGGGTTTGCCGGGTCAACCGGG
TCCGAAGGGTGATCGCGGTCCGAAAGGCCTGCCGGGTCCGCAGGGCTTACGTGGTCCG
AAGGGCGACAAA (SEQ ID NO: 9)

3) C8c amino acid sequence:

gKpgvtgfpgpqgplgKpgapgEpgpqgpigvpgvqgppgip (SEQ ID NO: 4)
gKpgvtgfpgpqgplgKpgapgEpgpqgpigvpgvqgppgip

gKpgvtgfpgpqgplgKpgapgEpgpqgpigvpgvqgppgip
gKpgvtgfpgpqgplgKpgapgEpgpqgpigvpgvqgppgip
gKpgvtgfpgpqgplgKpgapgEpgpqgpigvpgvqgppgip
gKpgvtgfpgpqgplgKpgapgEpgpqgpigvpgvqgppgip

(The amino acid sequence of the repeating unit of C8c is shown as SEQ ID NO: 4, with a repetition number of 6; the amino acid sequence of C8c is shown as SEQ ID NO: 5.)

Nucleotide sequence:

GGAAAACCCGGGGTAACTGGTTTTCCGGGTCCGCAGGGTCCGCTGGGTAAACCGGGTG
CACCGGGTGAACCGGGCCCGCAAGGTCCTATTGGTGTGCCGGGCGTTCAGGGCCCACC
GGGTATTCCGGGCAAGCCGGGCGTGACGGGATTTCCGGGTCCGCAAGGTCCGTTAGGC
AAGCCGGGCGCTCCGGGTGAACCGGGTCCTCAAGGTCCGATCGGTGTGCCTGGCGTCC
AGGGTCCACCGGGTATCCCGGGCAAACCGGGCGTTACCGGTTTCCCGGGCCCTCAAGGT
CCGCTGGGTAAGCCGGGTGCGCCGGGCGAGCCAGGCCCACAGGGTCCGATTGGTGTGC
CGGGCGTGCAAGGTCCACCGGGCATTCCGGGCAAACCGGGCGTTACCGGCTTCCCCGG
TCCGCAGGGTCCGCTGGGCAAGCCGGGCGCGCCAGGTGAGCCGGGCCCTCAAGGTCCG
ATCGGGGTCCCAGGTGTTCAAGGCCCGCCTGGCATTCCGGGTAAACCGGGCGTTACCGG
CTTTCCGGGTCCGCAAGGTCCGTTGGGTAAACCGGGTGCCCCTGGCGAGCCGGGTCCG
CAGGGTCCCATCGGCGTGCCGGGTGTTCAGGGTCCGCCGGGCATCCCGGGTAAACCGG
GCGTGACCGGTTTCCCGGGTCCGCAGGGTCCGCTGGGTAAGCCGGGTGCGCCAGGCGA
ACCGGGCCCGCAGGGTCCCATCGGTGTTCCGGGTGTCCAGGGCCCACCGGGCATCCCG(
SEQ ID NO: 6)

4) C8d amino acid sequence:

gKpgqDgipgqpgfpggKgEqglpglpgppglp (SEQ ID NO: 10)
gKpgqDgipgqpgfpggKgEqglpglpgppglp
gKpgqDgipgqpgfpggKgEqglpglpgppglp
gKpgqDgipgqpgfpggKgEqglpglpgppglp
gKpgqDgipgqpgfpggKgEqglpglpgppglp
gKpgqDgipgqpgfpggKgEqglpglpgppglp

(The amino acid sequence of the repeat unit of C8d is shown as SEQ ID NO: 10, with a repeat number of 6; the amino acid sequence of C8d is shown as SEQ ID NO: 11.)

Nucleotide sequence:

GGGAAACCCGGACAAGACGGCATTCCGGGTCAACCGGGATTCCCGGGTGGCAAAGGTG
AACAAGGTTTGCCAGGCTTGCCAGGTCCGCCTGGCTTGCCGGGCAAACCGGGCCAGGA
TGGCATCCCGGGCCAACCGGGCTTTCCGGGCGGTAAGGGCGAACAGGGTTTACCGGGT
CTGCCAGGCCCACCGGGTCTGCCGGGCAAGCCGGGTCAAGATGGTATTCCGGGTCAGC
CGGGTTTTCCGGGTGGTAAAGGTGAGCAGGGTTTGCCGGGGCTGCCGGGTCCGCCAGG
CCTGCCGGGTAAACCGGGTCAGGATGGTATCCCGGGTCAACCGGGTTTCCCGGGCGGTA
AAGGCGAGCAGGGTCTGCCGGGCCTTCCGGGTCCTCCGGGCCTGCCGGGTAAGCCGGG
TCAGGACGGCATCCCGGGACAACCGGGCTTCCCGGGCGGTAAGGGTGAGCAGGGTCTG
CCGGGCCTGCCGGGTCCGCCTGGTCTCCCGGGCAAGCCGGGCCAAGACGGCATTCCGG
GCCAGCCTGGCTTTCCGGGTGGTAAAGGTGAACAGGGCCTGCCGGGCCTGCCGGGTCC
GCCAGGCCTGCCG(SEQ ID NO: 12)

5) C8e amino acid sequence:

gKpgfpgpKgDRgmggvpgalgpRgEKgpigapgiggppgEpglpgipgpmgppgaigfpgpKgEggivgpqgppgpKg
EpglqgfpgKpgflgEvgppgmRglpgpigpKgEagqKgvpglpgvpgllgpKgEpgipgDqglqgppgipgiggpsgpigp
pgipgpKgEpglpgppgfp (SEQ ID NO: 13)

gKpgfpgpKgDRgmggvpgalgpRgEKgpigapgiggppgEpglpgipgpmgppgaigfpgpKgEggivgpqgppgpKg
EpglqgfpgKpgflgEvgppgmRglpgpigpKgEagqKgvpglpgvpgllgpKgEpgipgDqglqgppgipgiggpsgpigp
pgipgpKgEpglpgppgfp

(The amino acid sequence of the repeat unit of C8e is shown as SEQ ID NO: 13, with a repeat number of 2; the amino acid sequence of C8e is shown as SEQ ID NO: 14.)

Nucleotide sequence:

GGAAAACCCGGGTTCCCGGGCCCGAAAGGTGACCGCGGTATGGGTGGTGTCCCGGGCG
CGCTGGGTCCGCGTGGTGAGAAAGGCCCAATCGGTGCCCCTGGCATTGGCGGCCCGCC
AGGCGAACCGGGCCTGCCGGGCATTCCGGGTCCTATGGGCCCGCCGGGCGCAATTGGTT
TCCCGGGCCCCAAGGGTGAGGGTGGTATCGTGGGCCCACAGGGTCCACCGGGTCCAAA
AGGTGAGCCGGGCCTTCAAGGTTTCCCGGGGAAACCGGGTTTTCTGGGTGAAGTTGGT
CCACCGGGTATGCGCGGTTTGCCGGGTCCCATCGGTCCGAAGGGCGAAGCGGGTCAGA
AAGGTGTGCCGGGCTTGCCGGGCGTGCCGGGCTTGCTGGGTCCGAAGGGCGAACCGGG
CATTCCGGGTGATCAGGGTTTACAAGGTCCGCCAGGCATCCCGGGCATCGGCGGTCCGA
GCGGTCCTATTGGCCCACCGGGTATCCCGGGCCCGAAAGGTGAACCGGGCCTGCCTGGT
CCGCCAGGATTTCCGGGTAAGCCGGGCTTTCCGGGGCCGAAGGGCGACCGTGGTATGG
GTGGTGTTCCGGGTGCGCTGGGTCCGCGTGGTGAAAAAGGTCCGATCGGCGCTCCGGG
CATCGGTGGTCCTCCGGGTGAACCGGGACTCCCGGGTATTCCGGGTCCGATGGGTCCGC
CGGGTGCGATTGGTTTTCCGGGTCCGAAAGGTGAAGGTGGTATTGTTGGTCCGCAGGGT
CCTCCAGGTCCGAAGGGCGAGCCGGGTCTGCAGGGTTTTCCGGGGAAACCGGGTTTCC
TGGGTGAGGTTGGTCCGCCGGGTATGCGTGGTTTACCGGGGCCGATCGGCCCGAAGGGC
GAGGCAGGTCAAAAAGGCGTCCCGGGTCTGCCTGGCGTGCCGGGTTTGCTGGGCCCGA
AGGGCGAGCCGGGCATTCCGGGCGATCAGGGCCTGCAAGGTCCGCCAGGCATTCCGGG
GATCGGTGGTCCGTCCGGTCCCATCGGCCCGCCTGGCATCCCGGGACCGAAGGGCGAG
CCGGGACTGCCGGGCCCGCCGGGCTTCCCG (SEQ ID NO: 15)

6) C8f amino acid sequence:

gKpgfpgpKgDRgmggvpgalgpRgEKgpigapgiggpppgEpglpgipgpmgppgaigfpgpKgEggivgpqgppgpKg
EpglqgfpgKpgflgEvgppgmRglpgpigpKgEagqKgvpglpgvpgllgpKgEpgipgDq (SEQ ID NO: 16)

gKpgfpgpKgDRgmggvpgalgpRgEKgpigapgiggpppgEpglpgipgpmgppgaigfpgpKgEggivgpqgppgpKg
EpglqgfpgKpgflgEvgppgmRglpgpigpKgEagqKgvpglpgvpgllgpKgEpgipgDq

(The amino acid sequence of the repeat unit of C8f is shown as SEQ ID NO: 16, with a repeat number of 2; the amino acid sequence of C8f is shown as SEQ ID NO: 17.)

Nucleotide sequence:

GGGAAACCCGGATTCCCGGGTCCAAAGGGTGACCGCGGTATGGGTGGCGTGCCGGGTG
CGCTGGGTCCGCGTGGTGAGAAAGGTCCGATTGGCGCTCCGGGCATCGGCGGCCCACC
GGGTGAACCTGGATTGCCGGGCATCCCGGGTCCGATGGGTCCGCCGGGAGCCATTGGTT
TTCCGGGTCCGAAGGGTGAAGGTGGTATCGTGGGCCCACAGGGTCCGCCTGGGCCGAA
GGGCGAACCGGGCCTGCAGGGTTTTCCGGGTAAACCGGGCTTTCTGGGTGAGGTTGGT
CCGCCGGGCATGCGTGGCCTGCCGGGCCCGATCGGTCCGAAGGGCGAAGCAGGTCAGA

AAGGCGTCCCGGGTCTGCCGGGCGTGCCGGGCTTGCTGGGCCCAAAAGGTGAGCCGGG
TATTCCGGGGGATCAGGGTAAACCGGGCTTCCCGGGTCCGAAGGGTGACCGTGGTATGG
GCGGTGTGCCGGGCGCGCTGGGTCCGCGTGGTGAAAAAGGCCCCATCGGCGCGCCAGG
CATCGGCGGCCCGCCGGGCGAGCCGGGCTTACCGGGTATCCCGGGCCCGATGGGTCCGC
CGGGTGCGATTGGTTTCCCGGGCCCAAAGGGCGAGGGTGGTATTGTTGGTCCACAGGGC
CCACCGGGCCCCAAGGGTGAACCGGGCCTGCAAGGTTTTCCGGGGAAACCGGGCTTCC
TCGGTGAAGTTGGTCCGCCGGGTATGCGCGGTCTGCCTGGCCCGATTGGCCCAAAGGGT
GAGGCTGGCCAAAAGGTGTTCCGGGCCTTCCGGGCGTCCCTGGTTTGCTGGGTCCGA
AGGGTGAGCCGGGTATCCCGGGTGATCAA (SEQ ID NO: 18)

7) C8g amino acid sequence:

gKpgfpgpKgDRgmggvpgalgpRgEKgpigapgiggpppgEp (SEQ ID NO: 19)
gKpgfpgpKgDRgmggvpgalgpRgEKgpigapgiggpppgEp
gKpgfpgpKgDRgmggvpgalgpRgEKgpigapgiggpppgEp
gKpgfpgpKgDRgmggvpgalgpRgEKgpigapgiggpppgEp
gKpgfpgpKgDRgmggvpgalgpRgEKgpigapgiggpppgEp
gKpgfpgpKgDRgmggvpgalgpRgEKgpigapgiggpppgEp

(The amino acid sequence of the repeat unit of C8g is shown as SEQ ID NO: 19, with a repeat number of 6; the amino acid sequence of C8g is shown as SEQ ID NO: 20.)

Nucleotide sequence:

GGGAAACCCGGATTCCCGGGTCCGAAGGGCGATCGCGGTATGGGCGGTGTCCCGGGAG
CTTTGGGTCCGCGTGGTGAGAAAGGTCCGATTGGTGCTCCGGGTATTGGTGGTCCACCG
GGCGAGCCGGGTAAACCGGGCTTCCCTGGTCCGAAGGGCGATCGCGGCATGGGTGGCG
TGCCGGGTGCGCTGGGTCCGCGTGGTGAAAAAGGTCCGATCGGCGCGCCAGGCATCGG
CGGACCGCCTGGCGAACCTGGCAAACCGGGATTCCCGGGCCCGAAGGGTGACCGCGGT
ATGGGCGGTGTTCCGGGTGCATTGGGCCCGCGTGGCGAGAAGGGTCCGATTGGTGCGC
CAGGGATCGGCGGTCCTCCGGGCGAGCCGGGCAAACCGGGCTTTCCGGGCCCGAAAGG
TGATCGTGGCATGGGTGGTGTGCCGGGTGCGCTGGGCCCGCGTGGTGAAAAGGGCCCG
ATCGGCGCCCCGGGCATTGGCGGTCCTCCGGGCGAGCCGGGCAAGCCGGGTTTTCCGG
GTCCCAAGGGTGACCGTGGTATGGGTGGTGTTCCGGGTGCGCTGGGTCCGAGAGGTGA
GAAAGGGCCGATCGGTGCCCTGGCATCGGCGGTCCGCCAGGCGAACCGGGGAAACCG
GGTTTTCCGGGCCCAAAAGGCGACCGCGGTATGGGTGGTGTTCCGGGTGCGCTGGGTCC
GCGTGGTGAAAAGGGCCCGATCGGCGCACCGGGCATTGGAGGCCCACCGGGCGAACCG
(SEQ ID NO: 21)

8) C8h amino acid sequence:

gpKgEggivgpqgppgpKgEpglqgfpgKpgflgEvgppgmR (SEQ ID NO: 22)
gpKgEggivgpqgppgpKgEpglqgfpgKpgflgEvgppgmR
gpKgEggivgpqgppgpKgEpglqgfpgKpgflgEvgppgmR
gpKgEggivgpqgppgpKgEpglqgfpgKpgflgEvgppgmR
gpKgEggivgpqgppgpKgEpglqgfpgKpgflgEvgppgmR
gpKgEggivgpqgppgpKgEpglqgfpgKpgflgEvgppgmR

(The amino acid sequence of the repeat unit of C8h is shown as SEQ ID NO: 22, with a repeat number of 6; the amino acid sequence of C8h is shown as SEQ ID NO: 23.)

Nucleotide sequence:

GGGCCCAAAGGAGAGGGCGGTATCGTGGGCCCACAGGGTCCGCCGGGCCCCAAGGGT
GAACCGGGCCTGCAAGGCTTTCCGGGCAAACCGGGTTTCCTTGGCGAGGTTGGCCCAC
CGGGTATGCGCGGTCCGAAAGGCGAGGGTGGCATCGTGGGTCCACAAGGTCCGCCTGG
TCCGAAGGGTGAACCGGGCCTGCAGGGTTTCCCGGGTAAACCTGGCTTTCTGGGTGAG
GTTGGTCCGCCGGGCATGCGTGGTCCGAAGGGCGAGGGTGGTATCGTGGGCCCTCAGG
GTCCGCCGGGTCCGAAGGGCGAGCCGGGTTTGCAAGGTTTTCCGGGCAAACCGGGCTT
TCTGGGTGAGGTTGGTCCGCCGGGCATGCGTGGTCCGAAGGGTGAGGGCGGTATTGTTG
GTCCGCAGGGCCCACCGGGCCCGAAGGGTGAACCGGGTTTACAAGGCTTCCCGGGTAA
ACCGGGCTTTCTGGGCGAAGTTGGCCCACCGGGTATGCGTGGTCCGAAAGGCGAAGGT
GGTATTGTGGGTCCGCAGGGCCCACCGGGTCCGAAGGGGGAACCGGGTTTGCAAGGCT
TCCCGGGCAAACCGGGATTCTTGGGCGAAGTGGGCCCGCCGGGGATGCGTGGTCCGAA
GGGTGAAGGCGGTATTGTCGGTCCGCAGGGTCCGCCGGGTCCAAAAGGCGAACCGGGC
CTGCAGGGCTTCCCGGGTAAACCGGGCTTCCTGGGTGAGGTCGGTCCGCCGGGCATGC
GC (SEQ ID NO: 24).

9) C8i amino acid sequence:

gpKgEagqKgvpglpgvpgllgpKgEpgipgDq (SEQ ID NO: 25)
gpKgEagqKgvpglpgvpgllgpKgEpgipgDq
gpKgEagqKgvpglpgvpgllgpKgEpgipgDq
gpKgEagqKgvpglpgvpgllgpKgEpgipgDq
gpKgEagqKgvpglpgvpgllgpKgEpgipgDq
gpKgEagqKgvpglpgvpgllgpKgEpgipgDq

(The amino acid sequence of the repeat unit of C8i is shown as SEQ ID NO: 25, with a repeat number of 6; the amino acid sequence of C8i is shown as SEQ ID NO: 26.)

Nucleotide sequence:

GGGCCCAAAGGAGAGGCGGGTCAGAAAGGTGTGCCAGGCCTGCCAGGCGTTCCGGGT
TTGCTGGGTCCGAAGGGTGAACCGGGTATCCCGGGCGATCAGGGTCCGAAAGGTGAGG
CAGGTCAAAAAGGCGTGCCGGGCCTGCCGGGTGTGCCGGGCTTGCTTGGCCCGAAGGG
TGAACCGGGCATCCCGGGAGATCAAGGTCCTAAAGGCGAAGCTGGCCAAAAAGGCGTC
CCAGGCCTGCCGGGCGTGCCGGGTTTGCTGGGTCCGAAAGGCGAGCCGGGTATCCCGG
GCGACCAGGGTCCGAAGGGTGAAGCCGGTCAGAAAGGTGTTCCGGGCCTGCCGGGCG
TGCCGGGTTTGCTGGGCCCAAAGGGCGAGCCGGGCATTCCGGGTGACCAGGGTCCGAA
GGGCGAAGCGGGTCAGAAAGGCGTTCCGGGCCTGCCGGGTGTTCCGGGTCTGCTCGGT
CCGAAGGGTGAGCCGGGCATTCCGGGCGACCAAGGTCCGAAGGGCGAGGCGGGTCAG
AAAGGTGTTCCGGGTCTGCCTGGCGTCCCAGGTTTACTGGGTCCGAAGGGTGAACCGG
GTATTCCGGGCGATCAA (SEQ ID NO: 27)

10) C8j amino acid sequence:

gKpgvaglhgppgKpgalgpqgqpglpgppgppgppgpp (SEQ ID NO: 28)
gKpgvaglhgppgKpgalgpqgqpglpgppgppgppgpp
gKpgvaglhgppgKpgalgpqgqpglpgppgppgppgpp
gKpgvaglhgppgKpgalgpqgqpglpgppgppgppgpp
gKpgvaglhgppgKpgalgpqgqpglpgppgppgppgpp
gKpgvaglhgppgKpgalgpqgqpglpgppgppgppgpp

(The amino acid sequence of the repeat unit of C8j is shown as SEQ ID NO: 28, with a repeat number of 6; the amino acid sequence of C8j is shown as SEQ ID NO: 29.)

Nucleotide sequence:

GGGAAACCCGGAGTCGCCGGTTTGCACGGCCCACCGGGCAAACCGGGCGCTTTGGGCC
CGCAGGGCCAACCGGGCCTGCCAGGCCCACCGGGCCCGCCGGGCCCACCGGGTCCGCC
GGGTAAGCCGGGGGTGGCCGGTCTTCACGGCCCCCCGGGTAAACCGGGAGCTCTGGGT
CCTCAAGGTCAACCTGGCCTGCCGGGTCCGCCGGGCCCGCCGGGCCCACCGGGTCCGC
CGGGCAAGCCGGGCGTGGCAGGTCTGCACGGTCCGCCGGGGAAACCGGGTGCGCTGG
GTCCGCAAGGTCAACCGGGTTTACCGGGTCCGCCGGGTCCGCCGGGCCCACCGGGTCC
GCCGGGCAAGCCGGGCGTTGCAGGTTTGCACGGCCCGCCAGGCAAACCGGGTGCGCTG
GGTCCGCAGGGTCAGCCGGGCCTGCCGGGCCCGCCGGGCCCGCCAGGTCCGCCGGGCC
CGCCGGGTAAACCGGGTGTGGCGGGTCTGCACGGTCCGCCGGGCAAGCCTGGCGCGCT
GGGTCCGCAGGGTCAGCCGGGTCTGCCGGGCCCGCCTGGGCCGCCCGGTCCGCCGGGT
CCGCCTGGCAAGCCGGGTGTTGCGGGTCTGCATGGTCCGCCGGGCAAGCCGGGTGCGC
TGGGCCCGCAGGGTCAGCCGGGCTTGCCGGGTCCGCCGGGCCCGCCGGGTCCACCGGG
CCCGCCA (SEQ ID NO: 30)

2. [0196] Each of the aforementioned encoding nucleotide sequences was commercially synthesized. Subsequently, each of the above encoding nucleotide sequences (a collagen-processing enzyme cleavage site with the amino acid sequence of ENLYFQ and the nucleotide sequence of GAAAACCTGTATTTCCAG was added at the 5' end) was inserted between the KpnI and XhoI cleavage sites of the pET-28a-Trx-His expression vector to generate a recombinant expression plasmid.

3. The successfully constructed expression plasmid was transformed into E. coli competent cell BL21 (DE3). Specifically, the process was as follows: (1) taking out E. coli competent cells BL21 (DE3) from an ultra-low temperature refrigerator and placing them on ice. Once the cells were half - thawed, pipette 2 $\mu$l of the plasmid to be transformed into the E. coli competent cells BL21 (DE3) and gently mix 2 - 3 times. (2) Placing the mixture on ice in an ice bath for 30 minutes, then heat shocking in a water bath at 42°C for 45-90 s, and removing the mixture and placing it on ice in an ice bath for 2 minutes. (3) Transferring the mixture to a biosafety cabinet, adding 700 $\mu$l of liquid LB medium, and then incubating at 37°C and at 220 rpm for 60 min. (4) Taking 200 $\mu$l of the bacterial solution and evenly spreading it on a LB plate containing ampicillin sodium. (5) Incubating the plate in an incubator at 37°C for 15-17 h, until colonies of uniform size had grown.

4. 5-6 single colonies were picked from the LB plate with transformed cells and placed in shake flasks containing LB medium supplemented with antibiotic stock solution, followed by incubation in a shaker at 220 rpm and a constant temperature of 37°C for 7 hours. The cultured shake flask was then cooled to 16°C, and IPTG was added to induce expression for a specific period. The bacterial solution was dispensed into centrifuge bottles and centrifuged at 8000 rpm and 4°C for 10 minutes. The bacterial cells were collected, the bacterial cell weight was recorded, and samples (labeled "bacterial solution") were taken for electrophoresis detection.

5. The collected bacterial cells were resuspended in an equilibrium working solution (200 mM sodium chloride, 25 mM Tris, 20 mM imidazole at pH 8.0), and the bacterial solution was cooled to ≤ 15°C. The solution was homogenized by two rounds of high-pressure homogenization (samples were taken after each round and labeled "homogenization 1" and "homogenization 2", respectively), and the bacterial solution was collected after homogenization. The homogenized bacterial solution was divided into centrifuge bottles and centrifuged at 17,000 rpm and 4°C for 30 minutes. The supernatant was collected, and both the supernatant (labeled "Supernatant") and the pellets (labeled "Pellet") were taken for electrophoresis detection

6. Purifying and enzymatically digesting the recombinant type VIII humanized collagen. Specifically, the processes were as follows. (1) Crude purification: a. Washing the column material (Ni6FF, Cytiva) with water for 5 CVs. b. Equilibrating the column material with equilibrium solution (200 mM sodium chloride, 25 mM Tris, 20 mM imidazole, at pH 8.0) for 5 CVs. c. Loading: loading the obtained after centrifugation onto the column material until the liquid flowed through the column material completely, and then taking the flow through (labeled "Flow through") for electrophoresis detection. d. Washing impurity proteins: Adding 25 mL of washing solution (200 mM sodium chloride, 25 mM Tris, 20 mM imidazole) until the liquid flowed through completely, and taking the impurity-washing flow through (labeled "Impurity-washing") for electrophoresis detection. e. Collecting the target protein: adding 20 mL of eluate (200mM sodium chloride, 25mM Tris, 250mM imidazole, at pH 8.0), and collecting the flow-through liquid (marked: elution), detecting the protein concentration and calculating the protein amount, and performing electrophoresis detection. f. Washing the column material with 1 M imidazole working solution (labeled "Washing with 1 M"). g. Washing the column material with purified water. (2) Enzymatically digesting: according to the ratio of total protein amount to total TEV enzyme amount of 50: 1 (if not digested, consider increasing enzyme concentration, such as a total ratio of 20:1 or 5:1), adding TEV enzyme for digestion at 16°C for 4 h, and sampling for electrophoresis detection (labeled "After digestion"). Putting the enzymatically digested protein solution into a dialysis bag and dialyzing at 4°C for 2 h, then transferring it to a fresh dialysate for dialysis at 4°C overnight (labeled "Liquid exchange").

Fine purification (protein isoelectric point>8.0): a. equilibrating the column material (Capto Q, Cytiva): equilibrating the column material using solution A (20 mM Tris, 20 mM sodium chloride, at pH 8.0) at a flow rate of 10 ml/min. b. Loading: loading the sample at a flow rate of 5 ml/min and collecting the flow through (labeled "QFL"), and performing electrophoresis detection. c. Gradient eluting: Setting 0-15% solution B (20 mM Tris, 1 M sodium chloride, at pH 8.0) for 2 min (labeled as "Washing with 0-15% B solution") followed by holding for 3 CVs, 15-30% solution B for 2 min followed by holding for 3 CVs, 30-50% solution B for 2 min followed by holding 3 CVs, 50-100% solution B for 2 min followed by holding 3 CVs, respectively, collecting the peak and performing electrophoresis detection. d. Washing the column material. The protein was stored at 4°C.

Reverse nickel column (Ni6FF, Cytiva) purification (protein isoelectric point<8.0): a. Equilibrating the column: Solution A (20 mM Tris, 20 mM sodium chloride, 20 mM imidazole, pH 8.0) was used to equilibrate the column material for 5 CVs. b. Sample loading: The protein after enzymatic digestion and liquid exchange was added to the column until the liquid was completely drained, and then the flow-through sample was taken for electrophoresis detection (labeled as "Reverse Ni column"). c. Washing the column material with 1M imidazole working solution (20 mM Tris, 20 mM sodium chloride, 1M imidazole, pH 8.0) (labeled as "Washing with 1M"). d. The column material was cleaned with purified water. The protein was stored in a 4°C environment.

7. Concentration detection

An appropriate amount of sample was accurately measured, diluted 10-50 times with the eluent, and stirred thoroughly with a glass rod. The absorbance at 280nm was measured using a UV visible spectrophotometer, and the protein concentration was calculated according to the formula C (mg/ml) = A280 x absorbance coefficient x dilution factor (note: absorbance coefficient can be obtained based on amino acid sequence, and the absorbance value needs to be between 0.1 and 1).

The concentration detection results are as follows:

| Plasmid | Absorption coefficient | A280 | Dilution ratio | Concentration | Eluted protein volume | Protein quantity |
|---|---|---|---|---|---|---|
| C8a | 2.86 | 0.299 | 10 | 8.55 mg/ml | 20ml | 171.03mg |
| C8b | 2.43 | 0.292 | 10 | 7.10 mg/ml | 20ml | 141.91mg |
| C8c | 2.58 | 0.202 | 10 | 5.21 mg/ml | 20ml | 104.23mg |
| C8d | 2.30 | 0.237 | 10 | 5.45 mg/ml | 20ml | 109.02mg |
| C8e | 3.25 | 0.101 | 5 | 1.64 mg/ml | 20ml | 32.83mg |
| C8f | 2.79 | 0.260 | 5 | 3.63 mg/ml | 20ml | 72.54mg |
| C8g | 2.60 | 0.427 | 5 | 5.55 mg/ml | 20ml | 111.02mg |
| C8h | 2.66 | 0.321 | 1 | 0.85 mg/ml | 20ml | 17.08mg |
| C8i | 2.30 | 0.683 | 5 | 7.85 mg/ml | 20ml | 157.09mg |
| C8j | 2.44 | 0.486 | 1 | 1.19 mg/ml | 20ml | 23.72mg |

Protein expression levels are: C8a > C8i > C8b > C8g > C8d > C8c > C8f > C8e > C8j > C8h.

8. Electrophoretic detection

[0151] [0203] Specifically, the processes were as follows. 40 $\mu$L of solution was taken, and 10 $\mu$L of 5x protein loading buffer (250 mM Tris-HCl at pH 6.8, 10% SDS, 0.5% bromophenol blue, 50% glycerol, 5% $\beta$-mercaptoethanol) was added. The mixture was heated in boiling water at 100°C for 10 minutes, then 10 $\mu$L was loaded into each well of an SDS-PAGE protein gel. The gel was run at 80 V for 2 hours, stained for 20 minutes using Coomassie brilliant blue staining solution (0.1% Coomassie brilliant blue R-250, 25% isopropanol, 10% glacial acetic acid), and then decolorized using protein decolorization solution (10% acetic acid, 5% ethanol).

The electrophoresis detection results show that:

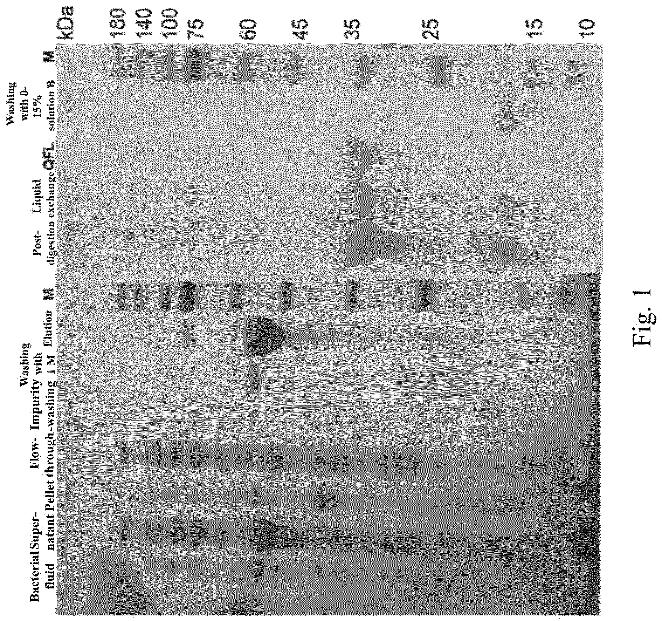

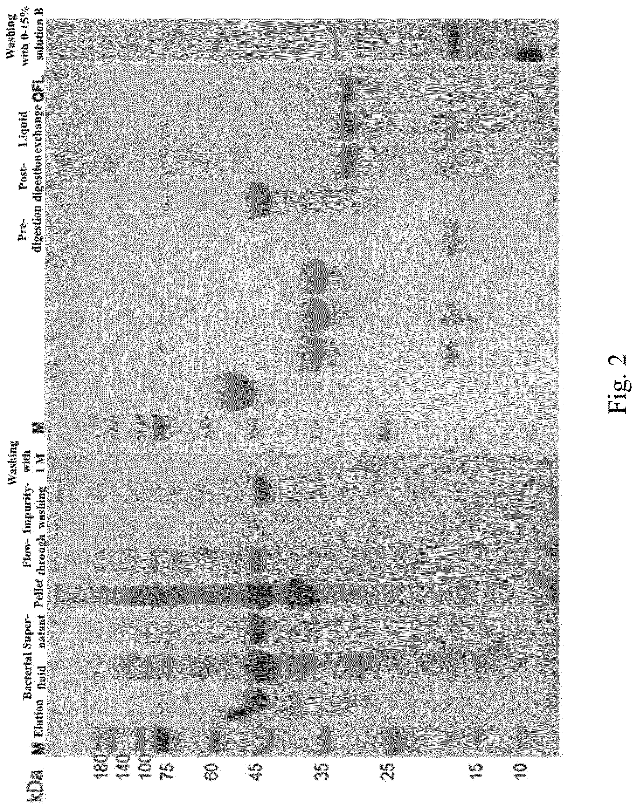

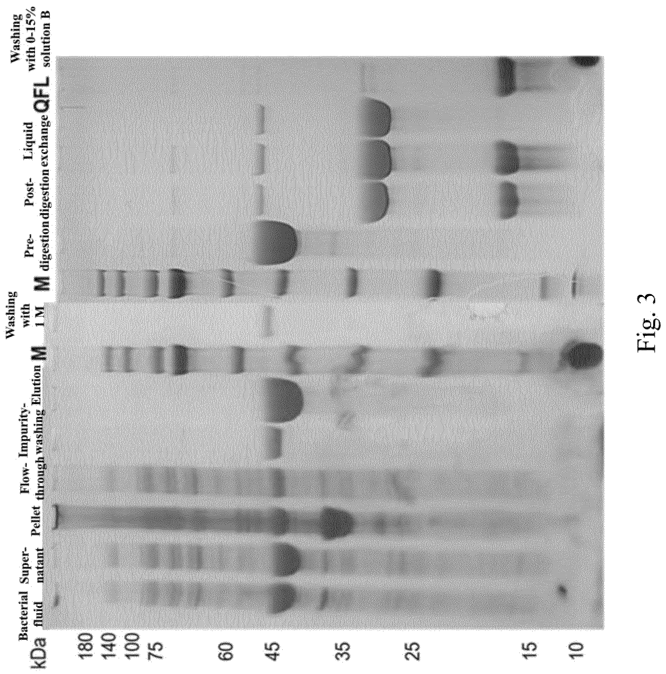

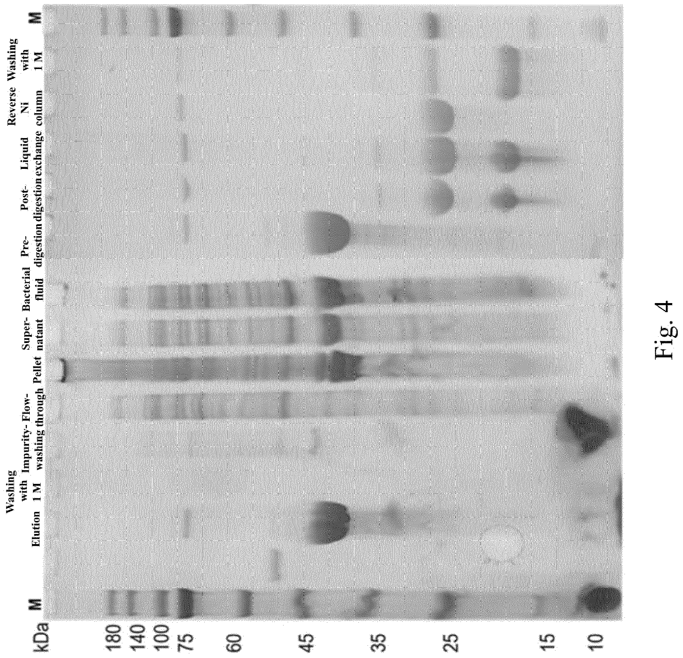

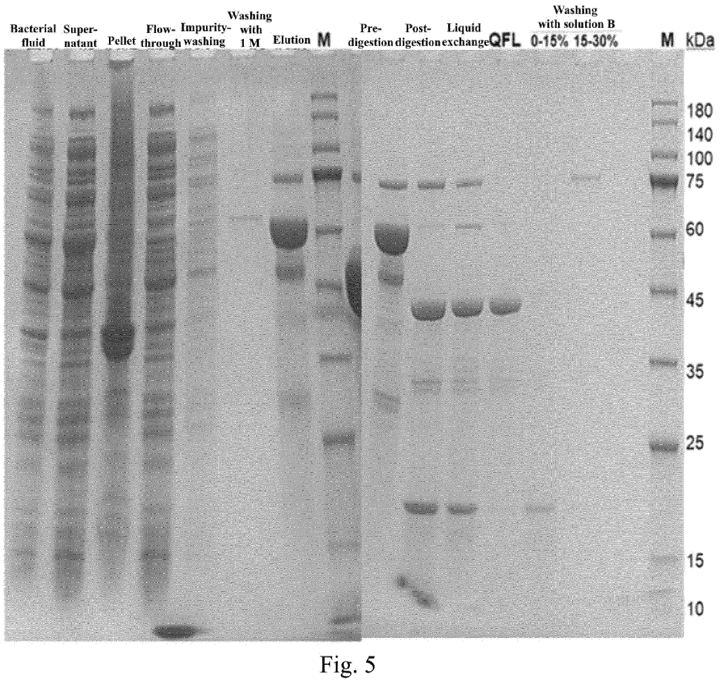

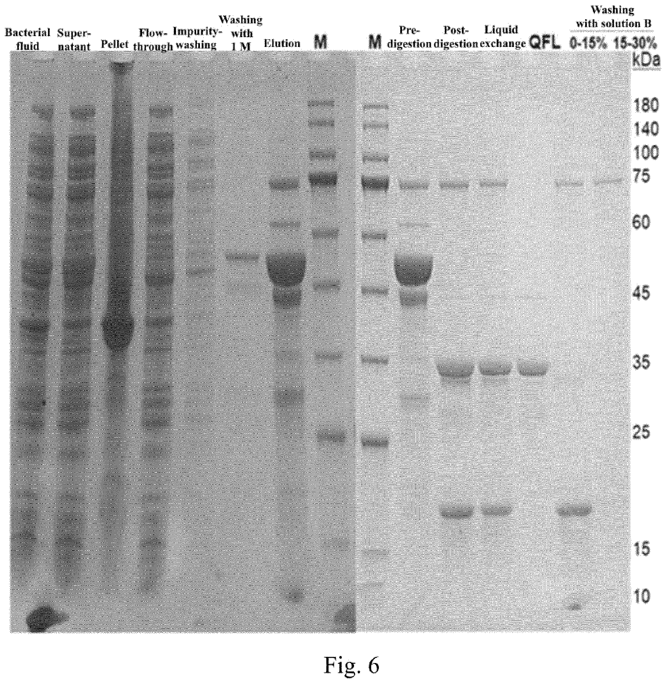

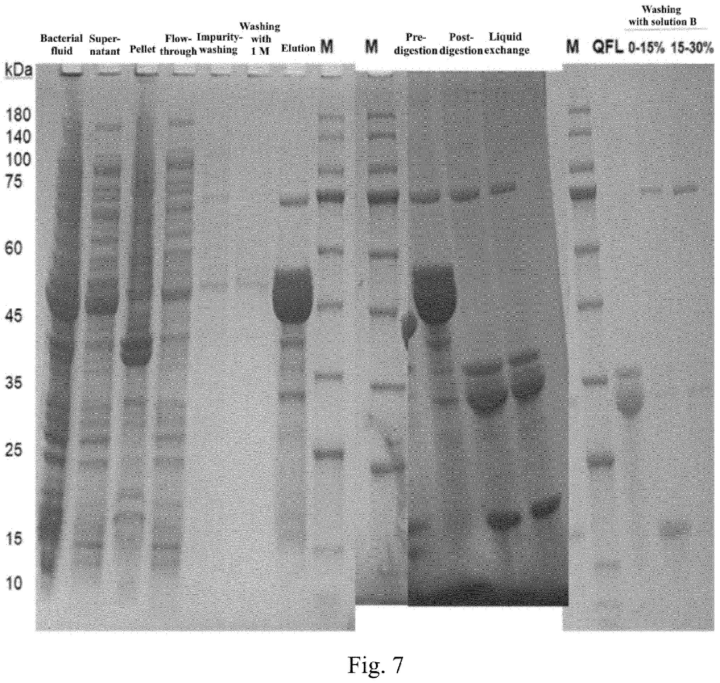

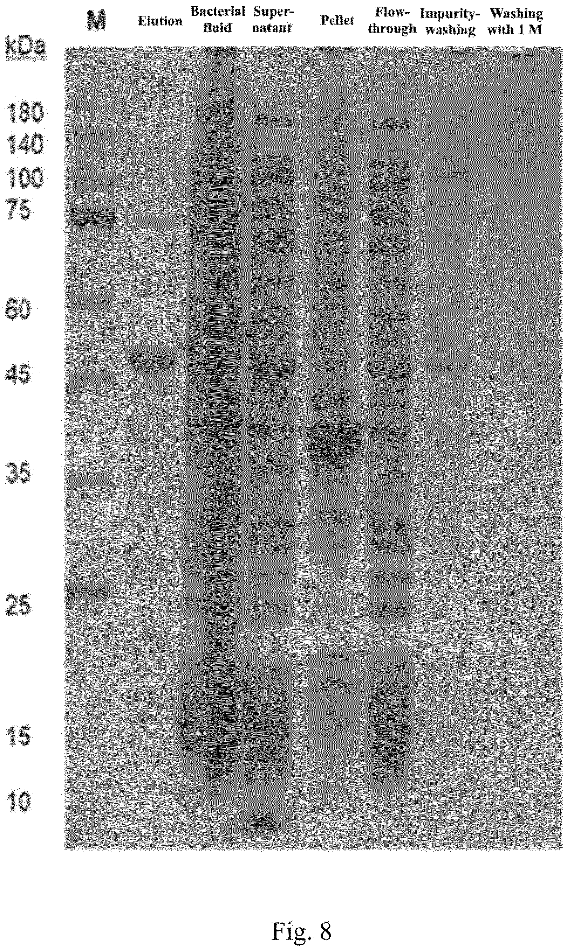

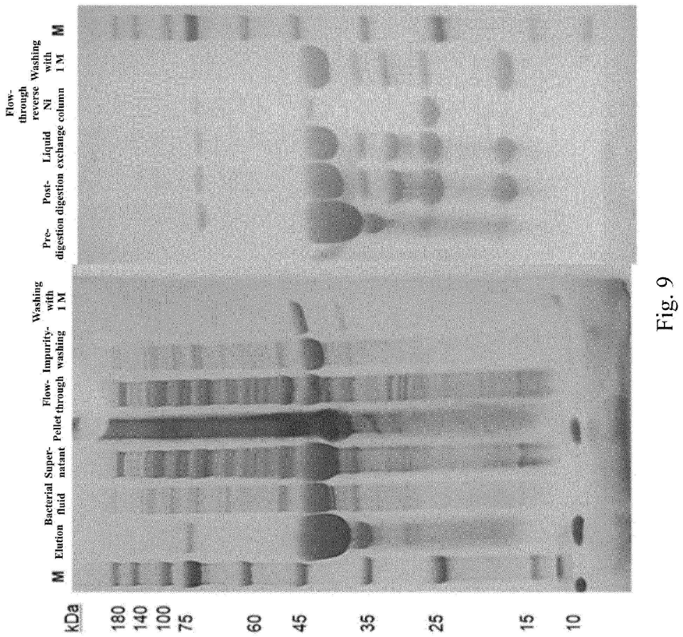

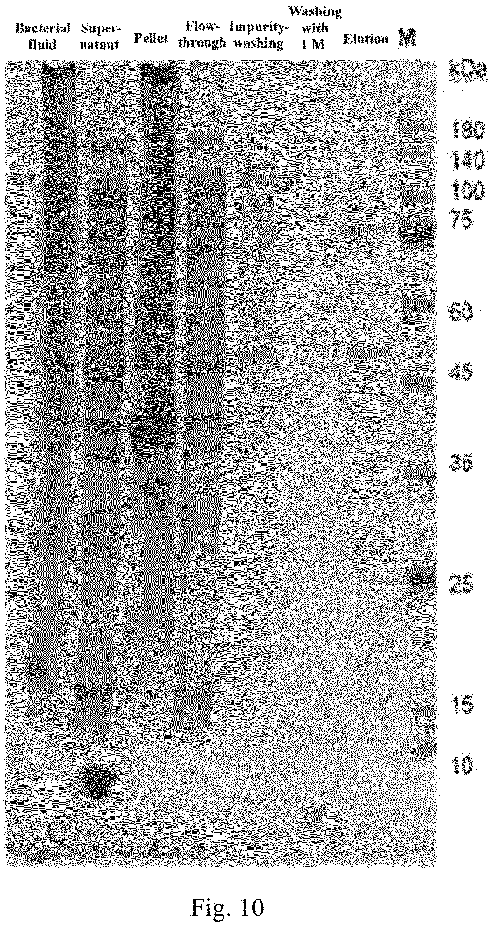

Fig. 1 shows the purification results of C8a. The yield of C8a is high, and the purity of the target protein is good after the fine purification. Fig. 2 shows the purification results of C8b. The crude target protein of C8b contains some impurity bands. When washed with 1M solution, some target proteins are eluted. After fine purification, the yield is low, but the purity of the target protein is good. Fig. 3 shows the purification results of C8c. The purity of the target protein of C8c is good. When digested with the enzyme at a ratio of 20:1, a small portion of the target protein is not digested. Fig. 4 shows the purification results of C8d. The yield of C8d is relatively high, but the impurity band at 75kDa is not removed after reverse nickel column purification. Fig. 5 shows the purification results of C8e. The crude target protein of C8e has impurity bands, and the yield is low. Fig. 6 shows the purification results of C8f. The crude target protein of C8f has a relatively large number of impurity bands. Fig. 7 shows the purification results of C8g. The yield of the crude C8g is relatively high, and the target protein appears as two bands. Fig. 8 shows the purification results of C8h. The crude yield of C8h is relatively low. Fig. 9 shows the purification results of C8i. For C8i, when digested with the enzyme at a ratio of 20:1, most of the protein is not cleaved, and even when the ratio is 5:1, it still remains undigested. Fig. 10 shows the purification results of C8j. The crude yield of C8j is relatively low.

The electrophoresis detection results showed that: 1. the crude purification yields for C8e, C8h, and C8j were low, and the bands of the target proteins were thin. 2. There was a distinct impurity band at 35kDa in the crude purified target protein of C8i, which could hardly be digested by the enzyme. 3. The target proteins of C8b and C8f had more impurity bands and lower yields after fine purification. 4. The yield of C8g was high, and the target protein appeared as two bands with low purity. 5. The yield of C8d was high, but there were more impurity bands and lower purity after using the reverse Ni column. 5. The yields of C8a and C8c were high, and the purity of the target proteins was good. C8a and C8c were selected for subsequent testing.

**Example 2: Mass spectrometry detection of recombinant type VIII humanized collagen**

Experimental methods

| Instrument name | Matrix assisted laser desorption ionization time-of-flight mass spectrometer MALDI-TOF/TOFUltraflextreme™, Brucker, Germany | | |
|---|---|---|---|
| Matrix | CHCA | Laser energy | 125 |
| Data retrieval software | Mascot | Retrieve species | All species |
| Retrieve database | Provide sequences as a library | | |

The protein samples were reduced with DTT and alkylated with iodoacetamide, followed by the addition of trypsin for enzymatic digestion overnight. The peptide fragments obtained after enzymatic digestion were desalted using C18ZipTip, mixed with the matrix $\alpha$-cyano-4-hydroxycinnamic acid (CHCA), and spotted onto a plate. Finally, the matrix-assisted laser desorption ionization-time of flight mass spectrometer (MALDI-TOF/TOF UlraflextremeTM, Bruker, Germany) was used for analysis (see Protein J. 2016;35:212-7 for peptide fingerprinting technology).

Data retrieval was performed using the MS/MS Ion Search page from the local Mascot website. The protein identification results were obtained based on the primary mass spectrometry of the peptide fragments generated after enzymatic digestion. Detection parameters: trypsin enzymatic digestion, allowing two missed cleavage sites. The alkylation of cysteine was set as a fixed modification, and the oxidation of methionine was set as a variable modification. The database used for identification was NCBprot.

Table 1: Molecular Weight and Corresponding Peptides Detected by Mass Spectrometry of Recombinant Type VIII Humanized Collagen C8a

| Start-End | Observed value | Mr(expt) | Mr(calc) | peptide |
|---|---|---|---|---|
| 1-21 | 1954.0164 | 1953.0091 | 1952.9457 | GKPGMPGMPGKPGAMGMPGAK |
| 262-288 | 2574.4059 | 2573.3986 | 2573.3061 | GEIGQKGEIGPMGIPGPQGPPGPHGLP |
| 268-288 | 1962.0511 | 1961.0438 | 1960.9829 | GEIGPMGIPGPQGPPGPHGLP |

Compared with the theoretical sequence, the coverage of detected peptide fragments is 100%, and the detection results are very reliable.

**Example 3: Biological Activity Detection of Recombinant Type VIII Humanized Collagen**

The methods for detecting collagen activity can be referred to in Juming Yao, Satoshi Yanagisawa, Tetsuo Asakura, Design, Expression and Characterization of Collagen-Like Proteins Based on the Cell Adhesive and Crosslinking Sequences Derived from Native Collagens, J Biochem. 136, 643-649(2004). The specific methods were performed as follows:

(1) The ultraviolet absorption method was used to detect the concentration of protein samples to be detected, including bovine type I collagen (National Institutes for Food and Drug Control, No. 380002), recombinant collagens C8a and C8c provided by the present disclosure.
Specifically, the ultraviolet absorption of the samples at 215nm and 225nm was measured respectively, and the protein concentrations were calculated using the empirical formula C ($\mu$g/mL) = 144 $\times$ (A215-A225). Note that the detection should be performed when A215< 1.5. The principle of this method is that the characteristic absorption of peptide bonds under far-ultraviolet light is detected, which is not affected by the chromophore content, with few interfering substances. The method is easy to operate, so it is suitable for the detection of human collagen and analogs thereof that do not develop color with Coomassie brilliant blue. (Refer to the reference Walker JM. The Protein Protocols Handbook, second edition. Humana Press. 43-45). After detecting the protein concentration, the concentration of all proteins to be tested was adjusted to 0.5 mg/mL with PBS.
(2) Sample preparation: the sample stock solution was used directly to conduct the experiment. The positive control human type I collagen (PC) was diluted to 1 mg/ml with D-PBS for future use; and the negative control was D-PBS buffer (NC).

(3) Coating: different concentrations of collagen and the positive control and the negative control were added to an ELISA plate in a volume of 100μL per well, and 5 replicate wells were set in each group, then incubated at 4 °C overnight.

(4) Blocking: The supernatant was discarded, and 100 μL of 1% BSA (heat-inactivated at 56°C for 30 minutes) was added, followed by incubation at 37°C for 60 minutes. The supernatant was discarded, and the plate was washed 3 times with D-PBS solution.

(5) Cell seeding: $10^5$ well-cultured 3T3/NIH cells resuspended in D-PBS were added to each well and incubated at 37°C for 120 minutes. Each well was washed 3 times with D-PBS solution.

(6) Detection: the absorbance at OD450 nm was detected using CCK8 detection kit (manufacturer Beyotime Biotech Inc., product catalog number C0038). The adherence degree of the cells was calculated according to the following formula. The adherence rate of cells can reflect the cell-adhesion ability of collagen. The higher the cell adhesion ability, the better the external environment can be provided to the cells in a short time to facilitate cell adherence.

$$p = (OD_1 - OD_0) / (OD_2 - OD_0)$$

where:

P: relative cell adhesion ratio;
$OD_1$: the average ultraviolet absorbance at 450 nm of all replicate wells of the tested collagen sample;
$OD_2$: Average ultraviolet absorbance at 450 nm for all replicate wells of the control collagen sample;
$OD_0$: Average ultraviolet absorbance at 450 nm for all replicate wells of the blank control group.

(7) Statistical analysis: The statistical difference between the target recombinant humanized collagen and the negative control was statistically analyzed by two-tailed t-test, where *, $P < 0.05$; * *, $P < 0.01$; * * *, $P < 0.001$.

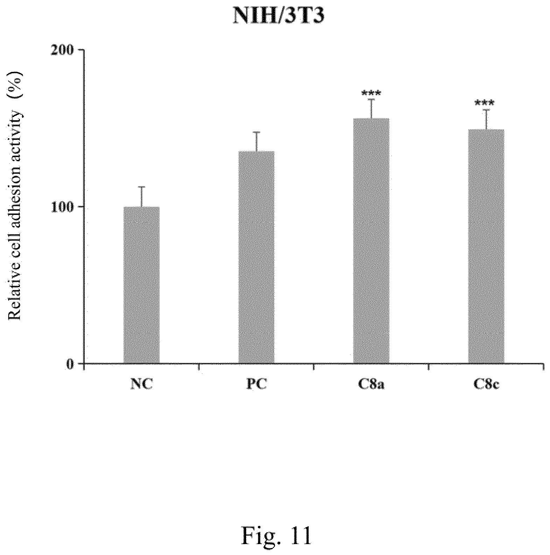

The results were shown in Fig. 11. As compared with the D-PBS group, the positive control had a significant effect in promoting cell adhesion, and the recombinant humanized collagens C8a and C8c also promote cell adhesion.

**Example 4: Analysis of recombinant type VIII humanized collagen protein by circular dichroism and ultraviolet scanning**

Experimental methods
(1) Instrument parameter setting

| | |
|---|---|
| Band width: | 1.0nm |
| Step | 1.0nm |
| Measurement range: | 190-260nm (far UV region scan)/250-340nm (near UV region scan) |
| Time-per-point | 0.5s |
| Repeat | 3 times |
| Cell Length | 10mm \| 0.5mm |
| Temperature | Room Temperature |

(2) Near and far ultraviolet scanning of standard samples

The scanning wavelength was set to 180-340nm for background testing and blank buffer testing, and then the circular dichroism of the 1mg/mL CSA standard solution in the 180 to 340nm range for near and far ultraviolet absorption was measured.

(3) Sample processing

Finely purified C8a and C8c protein samples were taken, and a 10 kD ultrafiltration concentration tube (Millipore) was used to concentrate the proteins to a protein concentration of 1mg/ml.

(4) Far Ultraviolet Scanning of Samples

The colorimetric dish was soaked in 2M HNO3 overnight, rinsed with deionized water, and air dried. The background was measured, and then the blank buffer was measured. Then, an appropriate amount of the test sample was added to the colorimetric dish and 190-260nm far ultraviolet scanning was performed according to the above parameters to collect data.

(5) Near UV Scanning of Samples

The colorimetric dish was soaked in 2M HNO3 overnight, rinsed with deionized water, and air dried. The background was collected, and then the blank buffer was collected. Then, an appropriate amount of the test sample was added to the colorimetric dish and near ultraviolet scanning at 250-340nm was performed according to the above parameters to collect data.

(6) Scanning spectrum processing

Subtract baseline and Smoothing processing were performed on all scanned spectra using Pro Data Viewer software.

Experimental results and analysis

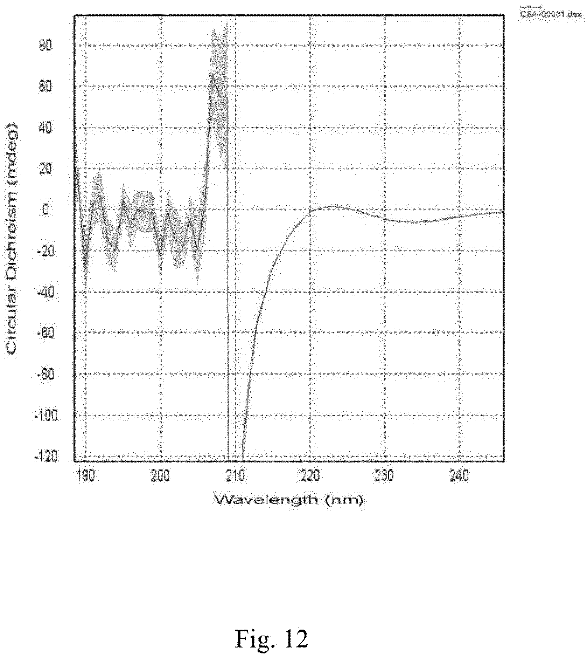

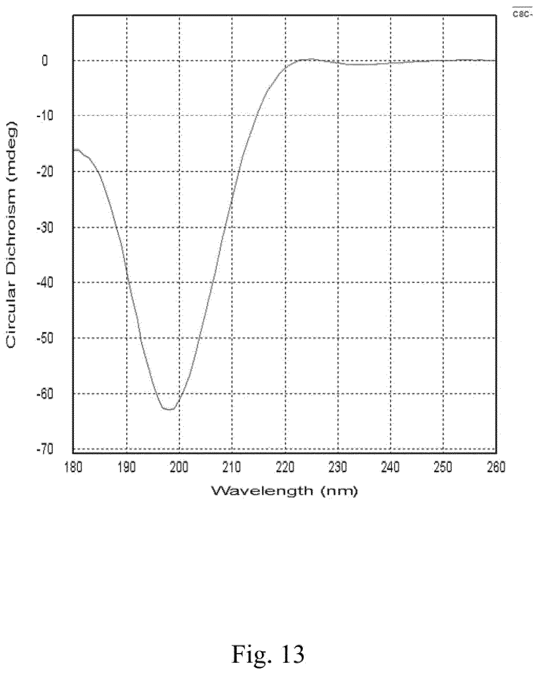

The results showed that both C8a and C8c had positive peaks at 221nm, indicating that the proteins had a triple helix structure (i.e., a common structural feature of active collagen). The results are shown in Figs. 12 and 13.

The above examples are preferred examples of the present disclosure, but the examples of the present disclosure are not limited by the above examples. Any other changes, modifications, substitutions, combinations, or simplifications made without departing from the spirit and principles of the present disclosure should be equivalent substitution methods and are included in the scope of protection of the present disclosure.

**Claims**

1. A collagen comprising one or more repeating units linked directly or via a linker, wherein the repeating units comprise an amino acid sequence selected from the group consisting of SEQ ID NO: 1, 4, 7, 10, 13, 16, 19, 22, 25 or 28, or a variant thereof, and wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence;

   preferably, wherein the repeating units are 2-50 repeating units, e.g., 2-45, 2-40, 2-35, 2-30, 2-25, 2-20, 2-15, 2-10 or 2-8 repeating units;
   preferably, wherein the linker comprises one or more amino acid residues, e.g., 1-10, 1-9, 1-8, 1-7, 1-6, 1-5, 1-4, 1-3 or 1-2 amino acid residues;
   preferably, wherein the mutations are selected from the group consisting of a substitution, an addition, an insertion or a deletion;
   preferably, wherein the substitution is a conservative amino acid substitution;
   preferably, the collagen is a recombinant collagen; preferably, the collagen is a recombinant type VIII collagen; preferably, the collagen is a human recombinant type VIII collagen;
   preferably, the collagen has cell adhesion activity, or has a triple helix structure or is in trimeric form.

2. The collagen according to claim 1, comprising an amino acid sequence selected from the group consisting of SEQ ID NO: 2, 5, 8, 11, 14, 17, 20, 23, 26, or 29, or a variant thereof, wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence;

   preferably, wherein the mutations are selected from the group consisting of a substitution, an addition, an insertion or a deletion;
   preferably, wherein the substitution is a conservative amino acid substitution.

3. A nucleic acid encoding the collagen according to claim 1 or 2,

preferably, wherein the nucleic acid comprises a codon optimized nucleotide sequence,
preferably, the nucleotide sequence is codon optimized for expression in *Escherichia coli;*
preferably, the nucleic acid comprises a nucleotide sequence selected from the group consisting of SEQ ID NO:3, 6, 9, 12, 15, 18, 21, 24, 27, or 30.

4. A vector comprising the nucleic acid according to claim 3,

preferably, wherein the vector comprises an expression control element, nucleotides of a purification tag and/or nucleotides of a leader sequence operably linked to the nucleic acid,
preferably, wherein the expression control element is selected from the group consisting of a promoter, a terminator or an enhancer;
preferably, wherein the purification tag is selected from the group consisting of a His tag, a GST tag, an MBP tag, a SUMO tag or a NusA tag;
preferably, the vector is an expression vector or a cloning vector, preferably pET-28a (+).

5. A host cell comprising the nucleic acid according to claim 3 or the vector according to claim 4; preferably, wherein the host cell is a eukaryotic cell or a prokaryotic cell; preferably, wherein the eukaryotic cell is a yeast cell, an animal cell and/or an insect cell, and/or the prokaryotic cell is an E. coli cell, for example E. coli BL21.

6. A composition comprising one or more of the collagen according to claim 1 or 2, the nucleic acid according to claim 3, the vector according to claim 4 and the host cell according to claim 5, preferably the composition is a kit; preferably, the composition is one or more of a biological dressing, a human bionic material, a plastic surgery and beauty material, an organoid culture material, a cardiovascular stent material, a coating material, a tissue injection filling material, an ophthalmic material, an obstetrics and gynecology biomaterial, a nerve repair and regeneration material, a liver tissue material and blood vessel repair and regeneration material, a 3D printed artificial organ biomaterial, a cosmetic raw material, a pharmaceutical excipient, and a food additive; preferably, wherein the composition is a composition for injection use, or oral use.

7. Use of the collagen according to claim 1 or 2, the nucleic acid according to claim 3, the vector according to claim 4, the host cell according to claim 5, and/or the composition according to claim 6 in one or more of a biological dressing, a human bionic material, a plastic surgery and beauty material, an organoid culture material, a cardiovascular stent material, a coating material, a tissue injection filling material, an ophthalmic material, an obstetrics and gynecology biomaterial, a nerve repair and regeneration material, a liver tissue material and blood vessel repair and regeneration material, a 3D printed artificial organ biomaterial, a cosmetic raw material, a pharmaceutical excipient, and a food additive.

8. A method of promoting cell adhesion, comprising the step of contacting the collagen according to claim 1 or 2, the nucleic acid according to claim 3, the vector according to claim 4, the host cell according to claim 5 and/or the composition according to claim 6 with a cell, preferably the cell is an animal cell, preferably a mammalian cell, preferably a human cell.

9. A method for performing plastic surgery or cosmetology, tissue injection filling, ophthalmic treatment, nerve repair or vascular repair on a subject in need thereof, comprising administering the collagen according to claim 1 or 2 to the subject, preferably orally or by injection; preferably, the subject has a disease or condition related to type VIII collagen deficiency, such as anterior segment hypoplasia, and preferably, the subject is human.

10. A method for producing the collagen according to claim 1 or 2, comprising:

(1) culturing the host cell according to claim 5 under a suitable culture condition;
(2) harvesting the host cell and/or culture medium containing the collagen; and
(3) purifying the collagen;
preferably, the host cell is an *Escherichia coli* cell, preferably an *Escherichia coli* BL21 (DE3) cell;
preferably, step (1) comprises culturing the *Escherichia coli* cell in LB medium and inducing expression through IPTG;
preferably, step (2) comprises harvesting *Escherichia coli* bacterial cells, resuspending them in an equilibrium working solution, homogenizing the *Escherichia coli* bacterial cells, preferably by high-pressure homogenization,

and isolating the supernatant; preferably, the equilibrium working solution comprises 100-500 mM sodium chloride, 10-50 mM Tris, 10-50 mM imidazole, pH7-9;

preferably, step (3) comprises crude purification and one or more of the following: enzymatic digestion, fine purification, and reverse nickel column purification;

preferably, the crude purification comprises purification of the supernatant by Ni-agarose gel column to obtain an eluant containing the target protein, wherein the eluant contains 100-500mM sodium chloride, 10-50mM Tris and 100-500mM imidazole, preferably pH7-9;

preferably, the enzymatic digestion comprises using TEV enzyme, preferably at a ratio of 10-100:1 of total protein to total amount of TEV enzyme for 2-8 hours;

preferably, the fine purification comprises gradient elution of the enzymatically digested product using a strong anion exchange chromatography column; preferably, gradient elution comprises 0-15% B solution for 1-5 min followed by holding for 3 column volumes; 15-30% B solution for 1-5 min followed by holding for 3 column volumes; 30-50% B solution for 1-5 min followed by holding for 3 column volumes; 50-100% B solution for 1-5 min followed by holding for 3 column volumes, where the B solution comprises 10-50 mM Tris, 0.5-5M sodium chloride, pH 7-9;

preferably, the reverse nickel column purification includes purification of the digested product on the Ni-agarose gel column; preferably, the eluent comprises 10-50 mM Tris, 10-50 mM sodium chloride, 0.5-5M imidazole, pH 7-9.

Fig. 1

Fig. 2

Fig. 3

Fig. 4

Fig. 5

Fig. 6

Fig. 7

EP 4 585 613 A1

Fig. 8

34

Fig. 9

Fig. 10

NIH/3T3

Fig. 11

Fig. 12

Fig. 13

## INTERNATIONAL SEARCH REPORT

| International application No. |
|---|
| **PCT/CN2023/121059** |

| A. | CLASSIFICATION OF SUBJECT MATTER |
|---|---|

C07K14/78(2006.01)i; C12N1/21(2006.01)i; C12N15/12(2006.01)i; C12N15/70(2006.01)i; A61L27/50(2006.01)i; A61L15/32(2006.01)i; A61K47/42(2017.01)i; A61K38/39(2006.01)i

According to International Patent Classification (IPC) or to both national classification and IPC

| B. | FIELDS SEARCHED |
|---|---|

Minimum documentation searched (classification system followed by classification symbols)

IPC: C07K; C12N; A61L; A61K

Documentation searched other than minimum documentation to the extent that such documents are included in the fields searched

Electronic data base consulted during the international search (name of data base and, where practicable, search terms used)

CJFD, CNTXT, ENTXTC, WFB, WFCCP, WFCD, WFCJ, WFCOA, WFCSTA, WFFCP, WFFD, WFFJ, WFFOA, WFS, WFSTR, PubMed, CNKI, ISI Web of Science, STN, GenBank: VIII型, VIII型胶原蛋白, 胶原蛋白, 重复, 重复单元, SEQ ID NOs: 1, 4, 7, 10, 13, 16, 19, 22, 25 and 28, collagen, repeat

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 113621052 A (SHANXI JINBO BIO-PHARMACEUTICAL CO., LTD.) 09 November 2021 (2021-11-09)<br>entire document | 1-10 |
| A | US 2002142391 A1 (KIVIRIKKO, K.I. et al.) 03 October 2002 (2002-10-03)<br>entire document | 1-10 |
| A | CN 110845603 A (INSTITUTE OF BIOPHYSICS, CHINESE ACADEMY OF SCIENCES) 28 February 2020 (2020-02-28)<br>entire document | 1-10 |
| A | CN 103122027 A (YANG, Xia et al.) 29 May 2013 (2013-05-29)<br>entire document | 1-10 |
| A | CN 115991764 A (HANGZHOU INSIGHTALE BIOTECHNOLOGY CO., LTD.) 21 April 2023 (2023-04-21)<br>entire document | 1-10 |

☑ Further documents are listed in the continuation of Box C.  ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **11 December 2023** | **19 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/121059** |

**C. DOCUMENTS CONSIDERED TO BE RELEVANT**

| Category* | Citation of document, with indication, where appropriate, of the relevant passages | Relevant to claim No. |
| --- | --- | --- |
| A | WO 2023024552 A1 (SHANXI JINBO BIO-PHARMACEUTICAL CO., LTD.) 02 March 2023 (2023-03-02)<br>entire document | 1-10 |
| A | CN 114940712 A (SHANXI JINBO BIO-PHARMACEUTICAL CO., LTD.) 26 August 2022 (2022-08-26)<br>entire document | 1-10 |
| A | 傅容湛等 (FU, Rongzhan et al.). "重组胶原蛋白的产业发展历程和生物医学应用前景展望 (Industrial Development and Biomedical Application Prospect of Recombinant Collagen)"<br>生物工程学报 《Chinese Journal of Biotechnology》,<br>Vol. 38, No. (9), 25 September 2022 (2022-09-25), 3228-3242<br>entire document | 1-10 |

Form PCT/ISA/210 (second sheet) (July 2022)

## INTERNATIONAL SEARCH REPORT

| International application No. |
| --- |
| **PCT/CN2023/121059** |

**Box No. I**      **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/CN2023/121059** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **9**
   because they relate to subject matter not required to be searched by this Authority, namely:

   Claim 9 relates to an ophthalmic treatment method, and does not comply with PCT Rule 39.1(iv). On the basis of the corresponding pharmaceutical use thereof,

   a search is carried out.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

# EP 4 585 613 A1

**INTERNATIONAL SEARCH REPORT**
**Information on patent family members**

International application No.

**PCT/CN2023/121059**

| Patent document cited in search report | | | Publication date (day/month/year) | Patent family member(s) | | | Publication date (day/month/year) |
|---|---|---|---|---|---|---|---|
| CN | 113621052 | A | 09 November 2021 | None | | | |
| US | 2002142391 | A1 | 03 October 2002 | None | | | |
| CN | 110845603 | A | 28 February 2020 | WO | 2021083072 | A1 | 06 May 2021 |
| | | | | EP | 4053160 | A1 | 07 September 2022 |
| | | | | EP | 4053160 | A4 | 01 November 2023 |
| | | | | JP | 2023504341 | A | 03 February 2023 |
| | | | | JP | 7300060 | B2 | 28 June 2023 |
| | | | | BR | 112022004900 | A2 | 28 June 2022 |
| | | | | US | 2022348639 | A1 | 03 November 2022 |
| | | | | KR | 20220058619 | A | 09 May 2022 |
| CN | 103122027 | A | 29 May 2013 | None | | | |
| CN | 115991764 | A | 21 April 2023 | None | | | |
| WO | 2023024552 | A1 | 02 March 2023 | None | | | |
| CN | 114940712 | A | 26 August 2022 | None | | | |

Form PCT/ISA/210 (patent family annex) (July 2022)

44

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310883772
- WO 9425612 A
- WO 2010039889 A

### Non-patent literature cited in the description

- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **RICE et al.** *EMBOSS: The European Molecular Biology Open Software Suite*, 2000
- **HUE et al.** *Journal of Bacteriology*, 1995, vol. 177, 3465-3471
- **GUO** ; **SHERMAN**. *Mol. Cellular Biol.*, 1995, vol. 15, 5983-5990
- **SIMONEN** ; **PALVA**. *Microbiological Reviews*, 1993, vol. 57, 109-137
- *Protein J.*, 2016, vol. 35, 212-7
- **JUMING YAO** ; **SATOSHI YANAGISAWA** ; **TETSUO ASAKURA**. Design, Expression and Characterization of Collagen-Like Proteins Based on the Cell Adhesive and Crosslinking Sequences Derived from Native Collagens. *J Biochem.*, 2004, vol. 136, 643-649
- **WALKER JM.** The Protein Protocols Handbook. Humana Press, 43-45